# EUROPEAN PATENT APPLICATION

(11) **EP 1 645 561 A1**
(43) Date of publication of application: **12.04.2006**
(21) Application number: 04733482.6
(22) Date of filing: 17.05.2004
(51) Int. Cl.: C07H 21/02, C07H 19/20, C07H 19/10

(54) **METHOD OF SYNTHESIZING CYCLIC BISDINUCLEOSIDE**

(30) Priority: 15.07.2003 JP 2003274389
(71) Applicant: Mitsui Chemicals, Inc., Tokyo 105-7117 (JP)
(72) Inventor: HAYAKAWA, Yoshihiro, Ichinomiya-shi, Aichi 491-0104 (JP)
(74) Representative: Stuart, Ian Alexander
(86) International application number: PCT/JP2004/007000
(87) International publication number: WO 2005/005450

(57) **Abstract**

A compound represented by Formula [2]: 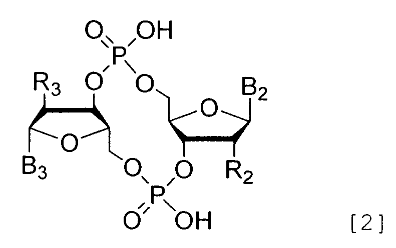 wherein R₂ and R₃ each independently represent a hydrogen atom, a halogen atom, a methoxy group, a 2-methoxyethoxy group or a hydroxyl group; and B₂ and B₃ each independently represent a nucleic acid base,
or a salt thereof can be synthesized from a compound represented by Formula [1]: 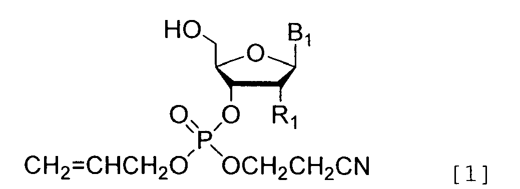 wherein R₁ represents a hydrogen atom, a halogen atom, a methoxy group, a 2-methoxyethoxy group, or a hydroxyl group substituted with a hydroxyl protecting group; and B₁ represents a nucleic acid base which may be protected.

## Description

### Technical Field

The present invention relates to a method for synthesizing a cyclic bis(3'→5')dinucleotide. More specifically, the invention relates to a method for efficiently synthesizing a cyclic bis(3'→5')dinucleotide with high yield through an intramolecular cyclization reaction of a dinucleotide.

A cyclic bis (3'→5') dinucleotide exhibits a physiological activity such as inhibition of cancerous cell division and is, therefore, a useful compound which is expected to be developed as a pharmaceutical product such as an anticancer agent. Thus, there is a demand on development of a practical method for synthesizing the compound.

### Background Art

Cyclic bis(3'→5')diguanylic acid (hereinafter, abbreviated to "cGpGp"), which is a member of cyclic bis(3'→5')dinucleotides, has been known for a long time as a functional substance regulating cellulose biosynthesis. Recently, it was also discovered that the substance has a biological activity of reducing cell division by increasing the amount of CD4 receptor when ingested into Molt4 and Jurkat cells. Due to the potential of the substance to inhibit cancerous cell division, a study for using the substance as an anticancer agent or clinical application thereof is anticipated. Therefore, there is an urgent need for mass production of a cyclic bis(3'→5')dinucleotide, which is represented by cGpGp.

Regarding the method for synthesizing cGpGp, methods using a p-chlorophenyl group as the protective group for the phosphoric acid moiety have already been reported (see Nature, 325, p. 279 (1987); and J. Biol. Chem., 265, p. 18993 (1990)). Furthermore, a case where cyclic bis(3'→5')diadenylic acid or cyclic bis(3'→5')diuridylic acid is synthesized by using a p-chlorophenyl group as the protective group for the phosphoric acid moiety has been reported (see Nucleosides & Nucleotides, 4(3), p. 377 (1985)).

However, the methods described in the aforementioned literatures [Nature, 325, p. 279 (1987)] and [J. Biol. Chem., 265, p. 18993 (1990)] are not practical because the synthesis yield of the dinucleotide, which is the starting material of the cyclization reaction, is as low as 75% (63%, if based on the substrate that is required in excess). Moreover, since it is necessary to separately carry out a reaction for removing the o-chlorophenyl group which is the protective group for the phosphoric acid moiety, and a reaction for removing the protective group for a nucleic acid base, multiple processes are required. Further, the deprotection processes necessarily take long reaction times such as 20 hours, 48 hours and 16 hours, respectively, thus the methods also not being practical in this point of view.

Likewise, the method described in the aforementioned literature [Nucleosides & Nucleotides, 4(3), p. 377 (1985)] is not practical because the synthesis yield of dinucleotide, which is the starting material for the cyclization reaction, is as low as 63%. Furthermore, since it is necessary to separately carry out a reaction for removing the p-chlorophenyl group which is the protective group for the phosphoric acid moiety and a reaction for removing the protective group for a nucleic acid base, multiple processes are required, and the yield is as low as 50 to 66%. Moreover, since either of the deprotection processes necessarily takes one whole day and night, the method is not practical.

Therefore, it is an object of the invention to provide a method for efficiently synthesizing cGpGp.

### Disclosure of the Invention

The inventors of the present invention examined the problems of conventional methods, such as low yield, multiple processes and long reaction time, and conducted active research on a protective group for the phosphoric acid moiety which can be applied to the synthesis of a cyclic bis(3'→5')dinucleotide, in order to provide an industrially practical synthesis method. As a result, they found that an allyl group and a 2-cyanoethyl group are useful as the protective group for the phosphoric acid moiety, thus completing the invention.

Thus, the present invention relates to a method for synthesizing a compound represented by Formula [2]: 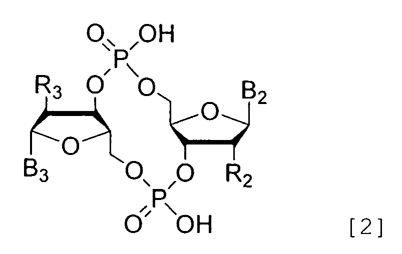 wherein R₂ and R₃ each independently represent a hydrogen atom, a halogen atom, a methoxy group, a 2-methoxyethoxy group or a hydroxyl group, and B₂ and B₃ each independently represent a nucleic acid base,
or a salt thereof from a compound represented by Formula [1]: 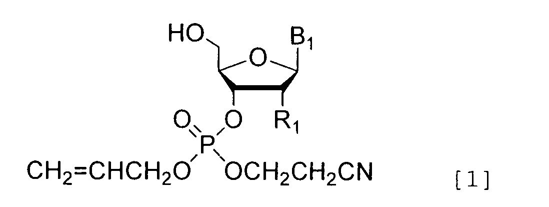 wherein R₁ represents a hydrogen atom, a halogen atom, a methoxy group, a 2-methoxyethoxy group, or a hydroxyl group substituted with a hydroxyl protecting group; and B₁ represents a nucleic acid base which may be protected.

According to the invention, a method for synthesizing a cyclic bis(3'→5')dinucleotide with high yield can be provided.

### Best Mode for Carrying Out the Invention

Hereinafter, the present invention will be described in detail.

The synthesis method of the invention is a method for obtaining a compound represented by Formula [2] or a salt thereof from a compound represented by Formula [1].

In Formula [1], R₁ represents a hydrogen atom, a halogen atom, a methoxy group, a 2-methoxyethoxy group, or a hydroxyl group substituted with a hydroxyl protecting group, and B₁ represents a nucleic acid base which may be protected.

Examples of the halogen atom include a fluorine atom, a chlorine atom, a bromine atom and an iodine atom.

With regard to the compound represented by Formula [1], the hydroxyl protecting group in R₁ can be any of conventionally used hydroxyl protecting groups and refers to a protective group which is removed by a chemical method such as hydrogenolysis, hydrolysis or photolysis.

The hydroxyl protecting group may be exemplified by a formyl group, an aliphatic acyl group, an aromatic acyl group, a silyl group, a silyloxymethyl group, an alkoxyalkyl group, an alkoxyalkyl group substituted with a silyl group, a halogenated alkyl group, an aralkyl group, an alkoxycarbonyl group, an aralkyloxycarbonyl group, an orthoester group or the like. Preferred hydroxyl protecting groups include the silyl group, the silyloxymethyl group, the alkoxyalkyl group substituted with a silyl group, and the orthoester group.

Examples of the silyl group include a trimethylsilyl group, a triethylsilyl group, a triisopropylsilyl group, a t-butyldimethylsilyl group and the like.

Examples of the silyloxymethyl group include a trimethylsilyloxymethyl group, a triethylsilyloxymethyl group, a triisopropylsilyloxymethyl group, a t-butyldimethylsilyloxymethyl group and the like.

Examples of the alkoxyalkyl group substituted with a silyl group include a 2-trimethylsilylethyloxymethyl group and the like. Examples of the orthoester group include a dimethoxymethyl group, a diethoxymethyl group, a bis(2-hydroxyethyloxy)methyl group, a bis(2-acetoxyethyloxy)methyl group and the like.

Examples of the hydroxyl group substituted with a hydroxyl protecting group include a trimethylsilyloxymethyloxy group, a triethylsilyloxymethyloxy group, a triisopropylsilyloxymethyloxy group, a t-butyldimethylsilyloxymethyloxy group, a 2-trimethylsilylethyloxymethyloxy group, a dimethoxymethyloxy group, a diethoxymethyloxy group, a bis(2-hydroxyethyloxy)methyloxy group, a bis(2-acetoxyethyloxy)methyloxy group and the like.

Examples of the nucleic acid base include natural or non-natural bases such as pyrimidine, purine, azapurine and deazapurine or the like, and these bases may be substituted with a halogen atom, an alkyl group having 1 to 4 carbon atoms, a haloalkyl group having 1 to 4 carbon atoms, an alkenyl group having 1 to 4 carbon atoms, a haloalkenyl group having 1 to 4 carbon atoms, an alkynyl group having 1 to 4 carbon atoms, an amino group, an alkylamino group having 1 to 4 carbon atoms, a hydroxyl group, a hydroxyamino group, an aminooxy group, an alkyloxy group having 1 to 4 carbon atoms, a mercapto group, an alkylthio group having 1 to 4 carbon atoms, an aryl group, an aryloxy group or a cyano group.

As the substituent, the halogen atom may be exemplified by chlorine, fluorine, iodine or bromine. The alkyl group may be exemplified by a methyl group, an ethyl group, a 1-propyl group or the like. The haloalkyl group may be exemplified by a fluoromethyl group, a difluoromethyl group, a trifluoromethyl group, a bromomethyl group, a bromoethyl group or the like. The alkenyl group may be exemplified by a vinyl group, an allyl group, a 3-butenyl group or the like. The haloalkenyl group may be exemplified by a bromovinyl group, a chlorovinyl group or the like. The alkynyl group may be exemplified by an ethynyl group, a propynyl group or the like. The alkylamino group may be exemplified by a methylamino group, an ethylamino group or the like. The alkyloxy group may be exemplified by a methoxy group, an ethoxy group or the like. The alkylthio group may be exemplified by a methylthio group, an ethylthio group or the like. The aryl group may be exemplified by a phenyl group; an alkylphenyl group having an alkyl group having 1 to 4 carbon atoms, such as a methylphenyl group or an ethylphenyl group; an alkoxyphenyl group having an alkyloxy group having 1 to 4 carbon atoms, such as a methoxyphenyl group or an ethoxyphenyl group; an alkylaminophenyl group having an alkylamino group having 1 to 4 carbon atoms, such as a dimethylaminophenyl group or a diethylaminophenyl group; a halogenophenyl group such as a chlorophenyl group or a bromophenyl group; or the like.

Specific examples of the pyrimidine base include cytosine, uracil, 5-fluorocytosine, 5-fluorouracil, 5-chlorocytosine, 5-chlorouracil, 5-bromocytosine, 5-bromouracil, 5-iodocytosine, 5-iodouracil, 5-methylcytosine, 5-methyluracil (thymine), 5-ethylcytosine, 5-ethyluracil, 5-fluorocytosine, 5-fluorouracil, 5-trifluoromethylcytosine, 5-trifluoromethyluracil, 5-vinyluracil, 5-(2-bromovinyl)uracil, 5-(2-chlorovinyl)uracil, 5-ethynylcytosine, 5-ethynyluracil, 5-propynyluracil, pyrimidin-2-one, 4-hydroxyaminopyrimidin-2-one, 4-aminooxypyrimidin-2-one, 4-methoxypyrimidin-2-one, 4-acetoxypyrimidin-2-one, 4-fluoropyrimidin-2-one, 5-fluoropyrimidin-2-one, 5-azacytosine and the like.

Specific examples of the purine base include purine, 6-aminopurine (adenine), 6-hydroxypurine, 6-fluoropurine, 6-chloropurine, 6-methylaminopurine, 6-dimethylaminopurine, 6-trifluoromethylaminopurine, 6-hydroxyaminopurine, 6-aminooxypurine, 6-methoxypurine, 6-acetoxypurine, 6-methylpurine, 6-ethylpurine, 6-trifluoromethylpurine, 6-phenylpurine, 6-mercaptopurine, 6-methylmercaptopurine, 6-aminopurine-1-oxide, 6-hydroxypurine-1-oxide, 2-amino-6-hydroxypurine (guanine), 2,6-diaminopurine, 2-amino-6-chloropurine, 2-amino-6-iodopurine, 2-aminopurine, 2-amino-6-marcaptopurine, 2-amino-6-methylmercaptopurine, 2-amino-6-hydroxyaminopurine, 2-amino-6-methoxypurine, 2-amino-6-benzoyloxypurine, 2-amino-6-acetoxypurine, 2-amino-6-methylpurine, 2-amino-6-cyclopropylaminopurine, 2-amino-6-phenylpurine, 2-amino-8-bromopurine, 6-cyanopurine, 6-amino-2-chloropurine (2-chloroadenine), 6-amino-2-fluoropurine (2-fluoroadenine) and the like.

Specific examples of the azapurine base and the deazapurine base include 6-amino-3-deazapurine, 6-amino-8-azapurine, 2-amino-6-hydroxy-8-azapurine, 6-amino-7-deazapurine, 6-amino-1-deazapurine, 6-amino-2-azapurine and the like.

The protected nucleic acid base refers to the nucleic acid base described above which has its amino group protected by a conventionally used amino protecting group.

The amino protecting group is not particularly limited, but examples thereof include acyl groups such as a formyl group, an acetyl group, a propionyl group, a butyryl group, an isobutyryl group, a valeryl group, a diphenylacetyl group, a phenoxyacetyl group, a benzoyl group and a toluoyl group; alkoxycarbonyl groups such as an allyloxycarbonyl group; alkyl groups such as an allyl group; amidine groups such as a dimethylaminomethylene group; and the like.

For the amino protecting group, it is desirable to select a protective group which can be removed under the same reaction conditions as in the removal of either a 2-cyanoethyl group or an allyl group, both of which are protective groups for the phosphoric acid ester of the compound represented by Formula [1].

The protective group is preferably a protective group that can be removed by ammonia, amines, or zero-valent palladium, divalent palladium complexes or divalent palladium salts described below.

Examples of such protective group include acyl groups such as a formyl group, an acetyl group, a propionyl group, a butyryl group, an isobutyryl group, a valeryl group, a diphenylacetyl group, a phenoxyacetyl group, a benzoyl group and a toluoyl group; amidine groups such as dimethylaminomethylene group; an allyl group; an allyloxycarbonyl group; and the like.

Among these, particularly preferred are the formyl group, the acetyl group, the isobutyryl group, the benzoyl group, the phenoxyacetyl group, the dimethylaminomethylene group, the allyl group and the allyloxycarbonyl group.

The protected nucleic acid base represented by B₁ may be exemplified by N⁴-acetylcytosine, N⁴-benzoylcytosine, N⁴⁻acetyl-5-fluorocytosine, N⁴-benzoyl-5-fluorocytosine, N⁴⁻acetyl-5-azacytosine, N⁴-benzoyl-5-azacytosine, N⁴⁻(phenoxyacetyl)cytosine, N⁴-(dimethylaminomethylene)cytosine, N⁴-(allyloxycarbonyl)cytosine, N⁴-acetyl-5-methylcytosine, N⁴-benzoyl-5-methylcytosine, N⁴-(phenoxyacetyl)-5-methylcytosine, N⁴-(dimethylaminomethylene)-5-methylcytosine, N⁴-(allyloxycarbonyl)-5-methylcytosine, N⁶-acetyladenine, N⁶⁻benzoyladenine, N⁶-(phenoxyacetyl)adenine, N⁶⁻(dimethylaminomethylene)adenine, N⁶⁻(allyloxycarbonyl)adenine, N⁶-acetyl-2-chloroadenine, N⁶⁻benzoyl-2-chloroadenine, N⁶-(phenoxyacetyl)-2-chloroadenine, N⁶-(dimethylaminomethylene)-2-chloroadenine, N⁶⁻(allyloxycarbonyl)-2-chloroadenine, N⁶-acetyl-2-fluoroadenine, N⁶-benzoyl-2-fluoroadenine, N⁶⁻(phenoxyacetyl)-2-fluoroadenine, N⁶⁻(dimethylaminomethylene)-2-fluoroadenine, N⁶⁻(allyloxycarbonyl)-2-fluoroadenine, N²,N⁶-diacetyl-2,6-diaminopurine, N²,N⁶-dibenzoyl-2,6-diaminopurine, N²,N⁶⁻bis(phenoxyacetyl)-2,6-diaminopurine, N²,N⁶⁻bis(dimethylaminomethylene)-2,6-diaminopurine, N²,N⁶⁻bis(allyloxycarbonyl)-2,6-diaminopurine, N²-acetylguanine, N²-benzoylguanine, N²-isobutyrylguanine, N²⁻(phenoxyacetyl)guanine, N²-(dimethylaminomethylene)guanine, N²-(allyloxycarbonyl)guanine, N²-(allyloxycarbonyl)-O⁶⁻allylguanine, N²-(allyloxycarbonyl)-N¹-allylguanine or the like.

The compound represented by Formula [1] can be synthesized with reference to the methods described in [Org. Lett., 3, p. 815 (2001) and Tetrahedron, 57, p. 8823 (2001)].

For example, regarding the method for synthesizing the compound represented by Formula [1], mention may be made of a method containing the steps of (1) reacting a compound represented by the following Formula (A) with 2-cyanoethanol in the presence of molecular sieves and a condensation activating agent, to obtain a compound represented by the following Formula (B); (2) subsequently, reacting the compound represented by Formula (B) with an oxidizing agent to obtain a compound represented by the following Formula (C); (3) subsequently, deprotecting the compound represented by Formula (C) to obtain the compound represented by Formula [1] (Reaction Scheme [1]).

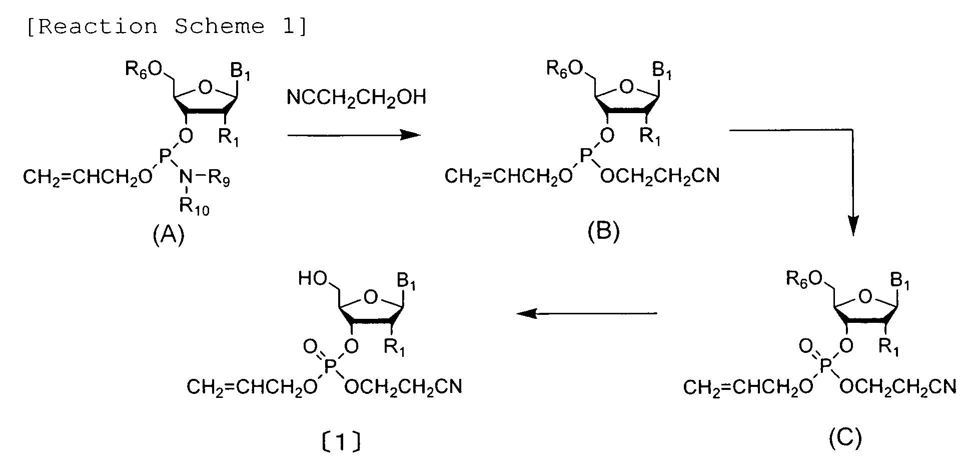

In Formula (A), R₁ and B₁ have the same meaning as defined for the above-described R₁ and B₁ in Formula [1]; R₆ represents a hydroxyl protecting group; R₉ and R₁₀ each independently represent an alkyl group having 1 to 4 carbon atoms, or R₉ and R₁₀ may form a ring containing the nitrogen atom to which R₉ and R₁₀ are bonded.

In the compound represented by Formula (A), the hydroxyl protecting group represented by R₆ may be those exemplified as the above-described hydroxyl protecting group for R₁ in Formula [1].

In the compound represented by Formula (A), when R₉ and R₁₀ are each an alkyl group having 1 to 4 carbon atoms, the R₉(R₁₀)N group may be exemplified by a diethylamino group, a di(n-propyl)amino group, an ethyl(n-propyl)amino group, an ethyl(isopropyl)amino group, a diisopropylamino group, a di(n-butyl)amino group, a diisobutylamino group or the like.

In the compound represented by Formula (A), when R₉ and R₁₀ form a ring containing the nitrogen atom to which R₉ and R₁₀ are bonded, the R₉ (R₁₀) N group forming such a ring may be exemplified by a pyrrolidino group, a piperidino group or the like.

In the compound represented by Formula (A), the R₉(R₁₀)N group is preferably a diisopropylamino group, a pyrrolidino group or a piperidino group.

R₁, R₆ and B₁ in Formula (B) and Formula (C) each have the same meaning as the above-described R₁, R₆ and B₁ of Formula (A).

The molecular sieve used in the aforementioned step (1) may be exemplified Molecular Sieve 3A and Molecular Sieve 4A.

Examples of the condensation activating agent used in the aforementioned step (1) include tetrazoles such as tetrazole, 5-methylthiotetrazole and 5-ethylthiotetrazole; imidazoles such as 4,5-dicyanoimidazole and 4,5-dichloroimidazole; imidazolim salts or benzimidazole salts such as N-phenylimidazole trifluoromethanesulfonate, imidazole tetrafluoroborate, N-phenylimidazole tetrafluoroborate, N-(p-acetylphenyl)imidazole trifluoromethanesulfonate, 2-phenylimidazole trifluoromethanesulfonate, 4-methylimidazole trifluoromethanesulfonate, 4-methylimidazole p-toluenesulfonate, 4-methylimidazole trifluoroacetate, 4-phenylimidazole trifluoromethanesulfonate, 4-phenylimidazole trifluoroacetate, benzimidazole tetrafluoroborate, N-methylbenzimidazole trifluoromethanesulfonate, 2-phenylbenzimidazole trifluoromethanesulfonate, 2-phenylbenzimidazole perchlorate, imidazolium perchlorate; and the like. Imidazolium perchlorate is particularly preferred.

In the aforementioned step (1), 2-cyanoethanol is typically used in an amount of 1 to 2 equivalents with respect to the compound represented by Formula (A).

The molecular sieves are typically used in an amount of 1% to 100% with respect to the weight of the compound represented by Formula (A). The preferred amount is 5% to 30%.

The condensation activating agent is typically used in an amount of 0.1 to 4 equivalents with respect to the compound represented by Formula (A). The preferred amount is 0.2 to 2 equivalents.

The reaction of the above-described step (1) generally employs a solvent. The solvent is not limited as long as it does not inhibit the reaction, but is preferably acetonitrile, dichloromethane, chloroform, THF, 1,4-dioxane or the like.

The reaction temperature for the above-described step (1) can be generally from -10°C to the boiling point of the solvent, but it is preferably in the range of 0°C to room temperature.

The compound represented by Formula (B) is used in the above-described step (2) without being isolated from the reaction mixture containing the compound represented by Formula (B) that is obtained from the above-described step (1) .

The oxidizing agent used in the aforementioned step (2) is not limited as long as it is conventionally used in, for example, oxidation of phosphite, but examples thereof include hydrogen peroxides such as aqueous hydrogen peroxide; peracids such as peracetic acid, perbenzoic acid and m-chloroperbenzoic acid; ketone peroxides such as acetone peroxide and 2-butanone peroxide; alcohol peroxides such as t-butyl hydroperoxide; persulfic acids such as potassium peroxysulfate; and the like.

The oxidizing agent is particularly preferably 2-butanone peroxide.

The above-mentioned oxidizing agent can be used as a solution in order to maintain the stability. The solvent used for preparing the solution is not particularly limited as long as it dissolves the oxidizing agent and does not inhibit oxidation, but preferred examples thereof include water; alcohols such as methanol and ethanol; ketones such as acetone and 2-butanone; aromatic hydrocarbons such as toluene, benzene and xylene; esters such as ethyl acetate, butyl acetate and dimethyl phthalate; and the like.

The oxidizing agent is typically used in an amount of 1 to 3 equivalents with respect to the compound represented by Formula (B).

The reaction of the aforementioned step (2) is generally carried out in a solvent. The solvent is not limited as long as it does not inhibit the reaction, but preferred examples include acetonitrile, dichloromethane, chloroform, THF, 1,4-dioxane and the like.

The reaction temperature for the above-described step (2) can be generally from -10°C to the boiling point of the solvent, and preferably in the range of 0°C to room temperature.

The compound represented by Formula (C) can be used in the above-described step (3) without being purified from the reaction mixture containing the compound represented by Formula (C) that is obtained from the step (1) and the step (2), after the reaction mixture being subjected to a general post-treatment such as liquid separation.

The R₆ group in Formula (C) is not particularly limited as long as it is a conventionally used hydroxyl protecting group, but it is preferred to use a protective group, such that the protective group represented by R₁ is not likely to be detached under the reaction conditions employed for removal of the R₆ group. Examples of such protective group include aralkyl groups such as 4,4'-dimethoxytrityl group; and silyl groups such as t-butyldimethylsilyl group, a bis(trimethylsiloxy)diphenylmethoxysilyl group and a bis(trimethylsiloxy)cyclododecyloxysilyl group.

Among these protective groups, 4,4'-dimethoxytrityl group is preferred.

The reaction conditions for deprotection in the aforementioned step (3) may vary depending on the R₆ group used.

When the R₆ group of Formula (C) is an aralkyl group, it is desirable to remove the protective group by using an acid. Examples of the acid used include sulfuric acid, hydrochloric acid, trifluoroacetic acid, perchloric acid, trichloroacetic acid, dichloroacetic acid and the like. Dichloroacetic acid is preferred.

When the R₆ group of Formula (C) is a silyl group, it is desirable to remove the protective group by using a fluoride. Examples of the fluoride used include pyridinium fluoride, tetrabutylammonium fluoride, potassium fluoride, sodium fluoride and the like. Tetrabutylammonium fluoride is preferred.

The acid is typically used in an amount of 1 to 40 equivalents with respect to the compound represented by Formula (C). The preferred amount is 10 to 30 equivalents.

The reaction of the aforementioned step (2) generally employs a solvent. The solvent is not limited as long as it does not inhibit the reaction, but preferred examples thereof include acetonitrile, dichloromethane, chloroform, THF, 1,4-dioxane and the like.

The reaction temperature for the aforementioned step (2) can be typically from -10°C to the boiling point of the solvent, and preferably in the range from 0°C to room temperature.

The reaction in each of the steps (1), (2), and (3) is generally carried out at an atmospheric pressure.

The compound represented by Formula [1] obtained from the step (3) can be separated and recovered from the reaction mixture by a known method such as, for example, column chromatography or crystallization.

R₂ and R₃ in Formula [2] each independently represent a hydrogen atom, a halogen atom or a hydroxyl group, while B₂ and B₃ each independently represent a nucleic acid base.

The halogen atom and the nucleic acid base may be those exemplified in the description for R₁ and B₁ of the above-described Formula [1].

The salt of the compound represented by Formula [2] is not limited as long as it consists of a base which generally forms a salt with phosphoric acid, but preferred examples thereof include the salts of alkali metals, alkaline earth metals and organic amines or the like. Examples of the alkali metal salt include lithium salts, sodium salts, potassium salts and the like. Examples of the alkaline earth metal salt include magnesium salts, calcium salts, barium salts and the like. Examples of the organic amine salt include tertiary amine salts such as triethylammonium salts and tri(n-butyl)ammonium salts; secondary amine salts such as diethylammonium salts and dicyclohexylammonium salts; primary amine salts such as ammonium salts, n-butylammonium salts and cyclohexylammonium salts; quaternary amine salts such as tetra(n-butyl)ammonium salts and trimethylbenzylammonium salts; and the like.

For example, a compound of Formula [2], in which R₂ and R₃ are each independently a hydrogen atom, a fluorine atom, a methoxy group, a 2-methoxyethoxy group or a hydroxyl group, or a salt thereof can be synthesized from a compound of Formula [1], in which R₁ is a hydrogen atom, a fluorine atom, a methoxy group, a 2-methoxyethoxy group or a t-butyldimethylsilyloxy group.

The method for synthesizing the compound represented by Formula [2] from the compound represented by Formula [1] is not particularly limited, but for example, mention may be made of a method of synthesizing the desired compound via compounds represented by Formula [5] and Formula [6], as shown in the following Reaction Scheme [2]:

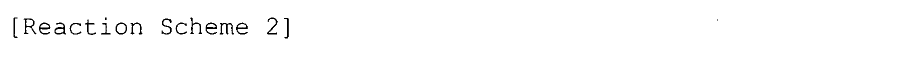

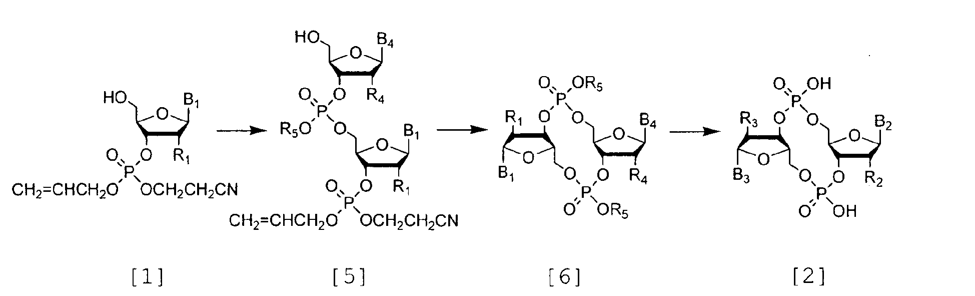

That is to say, mention may be made of a synthesis method containing the steps of deriving a compound represented by Formula [5] from the compound represented by Formula [1], subsequently deriving a compound represented by Formula [6] from the compound represented by Formula [5], and deriving the compound represented by Formula [2] from the compound represented by Formula [6].

For example, a compound of Formula [2], in which R₂ and R₃ are each independently a hydrogen atom, a fluorine atom, a methoxy group, a 2-methoxyethoxy group or a hydroxyl group can be synthesized from a compound of Formula [1], in which R₁ is a hydrogen atom, a fluorine atom, a methoxy group, a 2-methoxyethoxy group or a t-butyldimethylsilyloxy group, via compounds of Formula [5] and Formula [6], in which R₁ and R₄ are each independently a hydrogen atom, a fluorine atom, a methoxy group, a 2-methoxyethoxy group or a t-butyldimethylsilyloxy group.

In Formula [5], R₁ and R₄ each independently represent a hydrogen atom, a halogen atom, a methoxy group, a 2-methoxyethoxy group, or a hydroxyl group substituted with a hydroxyl protecting group; B₁ and B₄ each independently represent a nucleic acid base which may be protected; and R₅ represents an allyl group or a 2-cyanoethyl group. Furthermore, the hydroxyl group substituted with a hydroxyl protecting group and the nucleic acid base which may be protected may be those exemplified in the description for R₁ and B₁ of the above-described Formula [1].

In Formula [6], R₁, R₄, R₅, B₁ and B₄ have the same meanings as defined for R₁, R₄, R₅, B₁ and B₄ of the above-described Formula [5].

To specifically describe the method for synthesizing the compound represented by Formula [2] from the compound represented by Formula [1], mention may be made of, for example, a method for synthesizing the compound of Formula [2] from a compound represented by Formula [3] or Formula [4] and a compound represented by Formula [1], as illustrated in the following Reaction Scheme [3] or Reaction Scheme [4]:

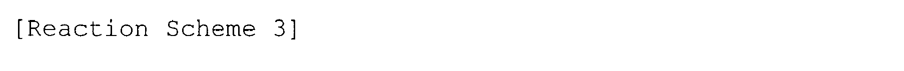

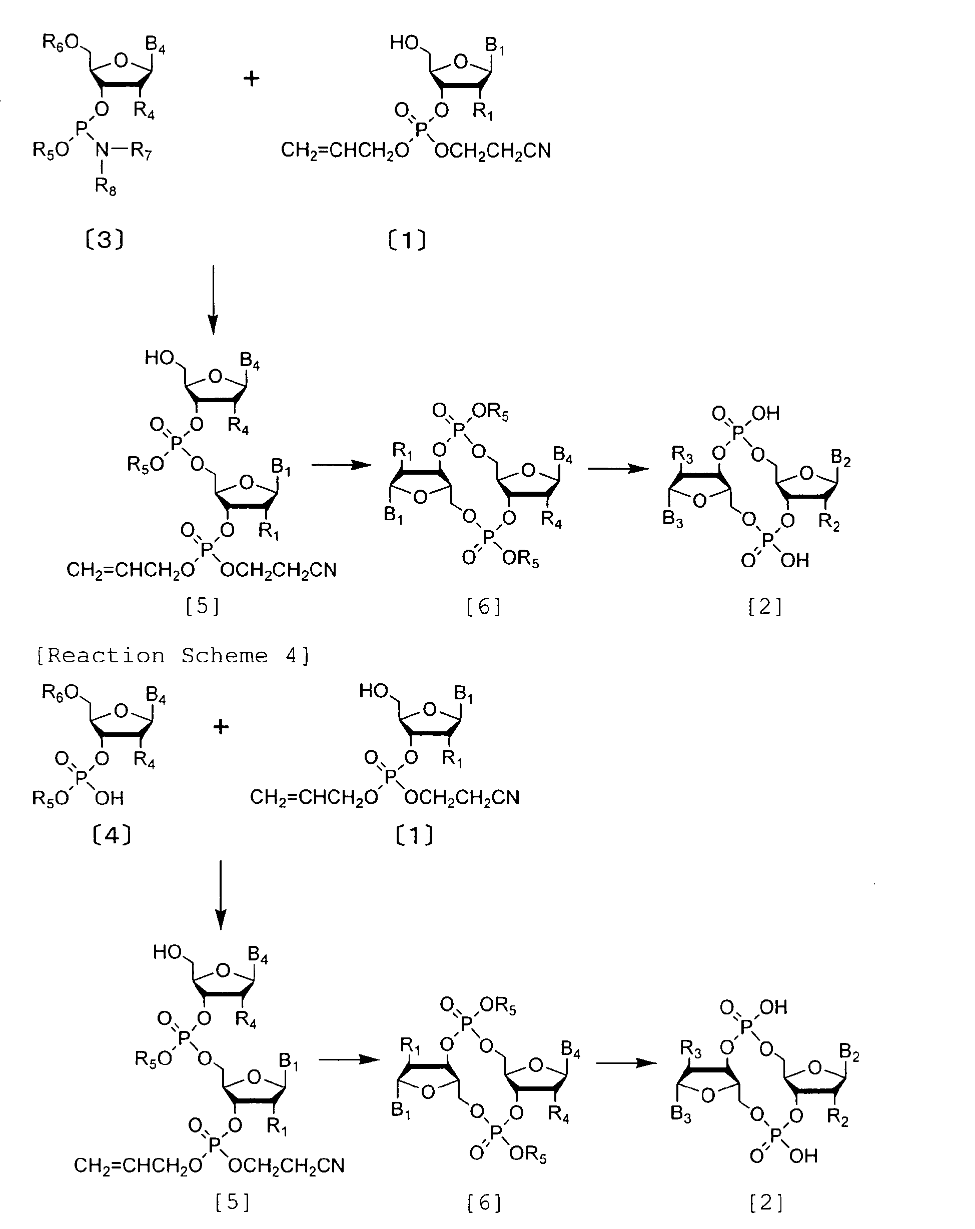

The method for synthesizing the compound represented by Formula [5] is not particularly limited as long as it is a method used for synthesizing general phosphoric acid ester compounds, but mention may be made of, for example, a method for condensing a compound represented by Formula [1] with a compound represented by Formula [3] and then oxidizing the condensation product, as illustrated in the following Reaction Scheme [5]; or a method for condensing a compound represented by Formula [1] with a compound represented by Formula [4], as illustrated in the following Reaction Scheme [6]:

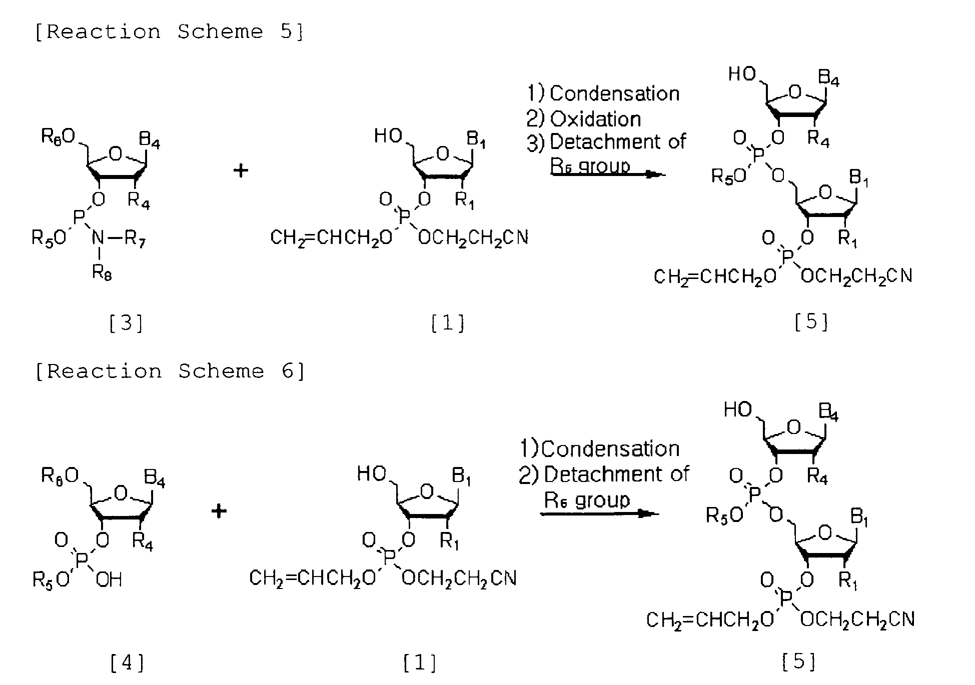

In Formula [3], B₄ and R₄ have the same meanings as defined for B₄ and R₄ of the above-described Formula [5]; R₆ has the same meaning as defined for R₆ of the above-described Formula (A); and R₇ and R₈ each independently represent an alkyl group having 1 to 4 carbon atoms, or R₇ and R₈ may be bonded to form a ring containing a nitrogen atom.

For the compound represented by Formula [3], when R₇ and R₈ are alkyl groups having 1 to 4 carbon atoms, the R₇(R₈)N group may be exemplified by a diethylamino group, a di(n-propyl)amino group, an ethyl(n-propyl)amino group, an ethyl(isopropyl)amino group, a diisopropylamino group, a di(n-butyl)amino group, a diisobutylamino group or the like.

For the compound represented by Formula [3], when R₇ and R₈ are bonded to form a ring containing a nitrogen atom, the R₇(R₈)N group forming such a ring may be exemplified by a pyrrolidino group, a piperidino group or the like.

For the compound represented by Formula [3], the R₇(R₈)N group is preferably a diisopropylamino group, a pyrrolidino group or a piperidino group.

In Formula [4], B₄, R₄ and R₆ have the same meanings as defined for B₄, R₄ and R₆ of the above-described Formula [3].

The condensation activating agent used for condensing the compound represented by Formula [1] with the compound represented by Formula [3], may be exemplified by tetrazoles such as tetrazole, 5-methylthiotetrazole or 5-ethylthiotetrazole; imidazoles such as 4,5-dicyanoimidazole or 4,5-dichloroimidazole; imidazolium salts or benzimidazole salts such as N-phenylimidazole trifluoromethanesulfonate, imidazole tetrafluoroborate, N-phenylimidazole tetrafluoroborate, N-(p-acetylphenyl)imidazole trifluoromethanesulfonate, 2-phenylimidazole trifluoromethanesulfonate, 4-methylimidazole trifluoromethanesulfonate, 4-methylimidazole p-toluenesulfonate, 4-methylimidazole trifluoroacetate, 4-phenylimidazole trifluoromethanesulfonate, 4-phenylimidazole trifluoroacetate, benzimidazole tetrafluoroborate, N-methylbenzimidazole trifluoromethanesulfonate, 2-phenylbenzimidazole trifluoromethanesulfonate, 2-phenylbenzimidazole perchlorate, imidazolium perchlorate; or the like.

The condensation activating agent is preferably imidazolium perchlorate.

The reaction temperature for the reaction using the condensation activating agent can be from -10°C to the boiling point of the solvent, and it is particularly preferably from 0°C to 40°C.

The oxidizing agent used for the oxidation of the product that is obtained by condensing the compound represented by Formula [1] with the compound represented by Formula [3], is not limited as long as it is generally used in the oxidation of phosphite, but examples thereof include hydrogen peroxides such as aqueous hydrogen peroxide; peracids such as peracetic acid, perbenzoic acid and m-chloroperbenzoic acid; ketone peroxides such as acetone peroxide and 2-butanone peroxide; alcohol peroxides such as t-butyl hydroperoxide; persulfates such as potassium peroxysulfate; and the like.

The oxidizing agent is preferably 2-butanone peroxide.

The aforementioned oxidizing agent can be used as a solution for the purpose of maintaining the stability.

The solvent used for conditioning the solution is not particularly limited as long as it dissolves the oxidizing agent and does not inhibit oxidation, but preferred examples thereof include water; alcohols such as methanol and ethanol; ketones such as acetone and 2-butanone; aromatic hydrocarbons such as toluene, benzene and xylene; esters such as ethyl acetate, butyl acetate and dimethyl phthalate; and the like.

The reaction temperature for oxidation can be from - 10°C to the boiling point of the solvent, and it is particularly preferably from 0°C to 40°C.

The reaction solvent for oxidation is not limited as long as it does not affect the oxidation reaction, but it is preferably an aprotic solvent, and particularly preferably acetonitrile, dichloromethane or THF.

The condensing agent used for the condensation of the compound represented by Formula [1] with the compound represented by Formula [4], may be exemplified by sulfonyl chlorides such as methanesulfonyl chloride, toluenesulfonyl chloride, 2,4,6-triisopropylbenzenesulfonyl chloride or mesitylene-2-sulfonyl chloride; sulfonyltetrazoles such as 1-toluenesulfonyltetrazole, 1-(mesitylene-2-sulfonyl)tetrazole or 1-(2,4,6-triisopropylbenzenesulfonyl)tetrazole; sulfonyltriazoles such as 3-nitro-1-toluenesulfonyl-1,2,4-triazole, 3-nitro-1-(mesitylene-2-sulfonyl)-1,2,4-triazole or 3-nitro-1-(2,4,6-triisopropylbenezenesulfonyl)-1,2,4-triazole; or the like. Triisopropylbenzenesulfonyl chloride is particularly preferred.

During the condensation, a base may be co-present. Examples of the base used therefor include triethylamine, ethyldiisopropylamine, pyridine, lutidine, imidazole, N-methylimidazole, N-methylbenzimidazole and the like. N-methylimidazole is particularly preferred.

The reaction temperature for condensation can be from - 10°C to the boiling point of the solvent, but is particularly preferably from 0°C to 40°C.

During the condensation, the reaction can be also carried out in the presence of a dehydrating agent in order to reduce the effect of moisture. The dehydrating agent can be those generally used for reactions, but molecular sieves are particularly preferred.

During the condensation, a reaction solvent is used in general. The reaction solvent is not limited as long as it does not affect the condensation, but it is preferably an aprotic solvent, and particularly preferably acetonitrile, dichloromethane or THF.

The R₆ group of Formula [3] and Formula [4] is not particularly limited as long as it is a conventionally used hydroxyl protecting group, but it is preferably a protective group such that the reaction conditions for the removal of the protective group R₆ do not cause detachment of the hydroxyl protecting group of R₁ or R₄, or the R₅ group.

Examples of such protective group R₆ include aralkyl groups such as 4,4'-dimethoxytrityl group; and silyl groups such as t-butyldimethylsilyl group, bis(trimethylsiloxy)diphenylmethoxysilyl group and bis(trimethylsiloxy)cyclododecyloxysilyl group.

Among these protective groups, 4,4'-dimethoxytrityl group is preferred.

The reaction conditions for the removal of the R₆ group may vary depending on the R₆ group used.

When the R₆ group is an aralkyl group, it is preferably removed by using an acid. Examples of the acid that can be used include sulfuric acid, hydrochloric acid, trifluoroacetic acid, perchloric acid, trichloroacetic acid, dichloroacetic acid and the like. Dichloroacetic acid is preferred.

When the R₆ group is a silyl group, it is preferably removed by using a fluoride. Examples of the fluoride that can be used include pyridinium fluoride, tetrabutylammonium fluoride, potassium fluoride, sodium fluoride and the like. Tetrabutylammonium fluoride is preferred.

The acid used for the deprotection is generally used in an amount of 1 to 40 equivalents with respect to the theoretical amount of generation of the compound represented by Formula [5], but the amount is preferably from 10 to 30 equivalents.

The reaction for the removal of the R₆ group generally involves the use of a solvent. The solvent is not limited as long as it does not inhibit the reaction, but it is preferably acetonitrile, dichloromethane, chloroform, THF, 1,4-dioxane or the like.

The reaction temperature for the removal of the R₆ group can be typically from -10°C to the boiling point of the solvent, but it is preferably in the range of 0°C to room temperature.

The method for synthesizing the compound represented by Formula [6] is not particularly limited, but mention may be made of, for example, a method in which, when the R₅ group of the compound represented by Formula [5] is a 2-cyanoethyl group, a cyclization reaction is carried out after removing an allyl group, whereas when the R₅ group of the compound represented by Formula [5] is an allyl group, a cyclization reaction is carried out after removing a 2-cyanoethyl group, as illustrated in the following Reaction Scheme [7]:

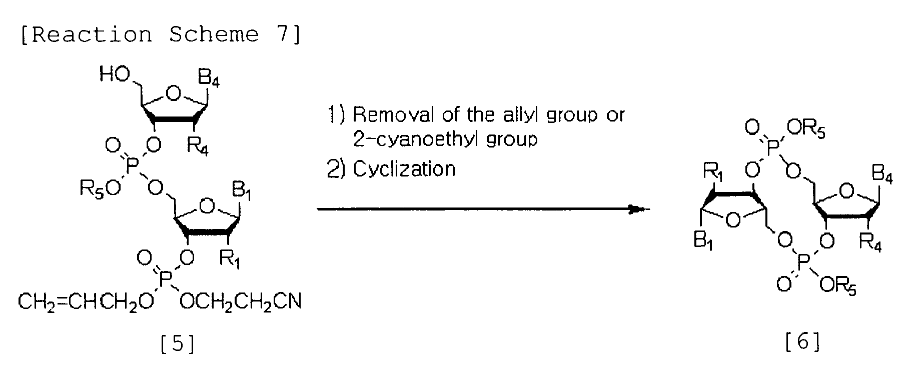

The reagent used for the removal of the 2-cyanoethyl group is not particularly limited, but for example, ammonia or amines can be used.

Examples of the amines include primary amines such as methylamine and ethylamine; secondary amines such as dimethylamine, diethylamine and piperidine; and tertiary amines such as trimethylamine, triethylamine and 1-methylpiperidine.

The reaction temperature for the deprotection with ammonia or amines can be from -10°C to the boiling point of the solvent used, and the reaction is preferably carried out at a temperature between room temperature and 70°C.

The reagent used for the removal of the allyl group is not particularly limited, but zero-valent palladium, divalent palladium complexes and divalent palladium salts can be used.

The zero-valent palladium, divalent palladium complex or divalent palladium salt is not particularly limited, but preferred examples thereof include palladium supports such as palladium carbon and palladium hydroxide carbon; acid palladium salts such as palladium acetate, palladium chloride and dichloro(1,5-cyclooctanediene)palladium; phosphine complexes such as tetrakis(triphenylphosphine)palladium and bis(tricyclohexylphosphirie)palladium; diketo complexes such as palladium 2,4-pentanedionate and tris(dibenzylideneacetone)dipalladium-chloroform; and the like.

The reaction temperature for the deprotection with the zero-valent palladium, divalent palladium complex or divalent palladium salt, can be from -10°C to the boiling point of the solvent used, and the reaction is preferably carried out at a temperature between 0°C and 50°C.

During the removal of the allyl group or 2-cyanoethyl group without removing the R₅ group in Formula [5], when R₅ of Formula [5] is an allyl group, R₁ or R₄ is a hydroxyl protecting group, and B₁ or B₄ is a protected nucleic acid base, any of the protective groups for R₁, R₄, B₁ and B₄ is preferably stable against the reaction conditions for removing a 2-cyanoethyl group. Furthermore, when R₅ of Formula [5] is a 2-cyanoethyl group, R₁ or R₄ is a hydroxyl protecting group, and B₁ or B₄ is a protected nucleic acid base, any of the protective groups for R₁, R₄, B₁ and B₄ is preferably stable against the reaction conditions for removing an allyl group.

When R₅ of Formula [5] is a 2-cyanoethyl group, examples of the hydroxyl protecting group for R₁ and R₄ in Formula [5] include acyl groups such as a formyl group, an acetyl group, a propionyl group, a butyryl group, an isobutyryl group, a valeryl group, a diphenylacetyl group, a phenoxyacetyl group, a benzoyl group and a toluoyl group; silyl groups such as a trimethylsilyl group, a triethylsilyl group, a triisopropylsilyl group and a t-butyldimethylsilyl group; silyloxymethyl groups such as trimethylsilyloxymethyl group, a triethylsilyloxymethyl group, a triisopropylsilyloxymethyl group and a t-butyldimethylsilyloxymethyl group; alkoxyalkyl groups substituted with a silyl group such as a 2-trimethylsilylethyloxymethyl group; orthoester groups such as a dimethoxymethyl group, a diethoxymethyl group, a bis(2-hydroxyethyloxy)methyl group and a bis(2-acetoxyethyloxy)methyl group; and the like.

When R₅ of Formula [5] is a 2-cyanoethyl group, examples of the protective group for the protected nucleic acid base of B₁ and B₄ of Formula [5] include acyl groups such as a formyl group, an acetyl group, a propionyl group, a butyryl group, an isobutyryl group, a valeryl group, a diphenylacetyl group, a phenoxyacetyl group, a benzoyl group and a toluoyl group; amidines such as a dimethylaminomethylene group; and the like.

When R₅ of Formula [5] is an allyl group, examples of the hydroxyl protecting group for R₁ and R₄ of Formula [5] include allyl groups such as an allyl group and an allyloxycarbonyl group; silyl groups such as a trimethylsilyl group, a triethylsilyl group, a triisopropylsilyl group and a t-butyldimethylsilyl group; silyloxymethyl groups such as a trimethylsilyloxymethyl group, a triethylsilyloxymethyl group, a triisopropylsilyloxymethyl group and a t-butyldimethylsilyloxymethyl group; alkoxyalkyl groups substituted with a silyl group such as a 2-trimethylsilylethyloxymethyl group; and the like.

When R₅ of Formula [5] is an allyl group, examples of the protective group for the protected nucleic acid base of B₁ and B₄ of Formula [5] include allyl groups such as an allyl group and an allyloxycarbonyl group.

The cyclization reaction can employ a general method for synthesizing phosphoric acid esters.

The condensing agent used in the cyclization reaction is not particularly limited, but preferred examples thereof include sulfonyl chlorides such as methanesulfonyl chloride, toluenesulfonyl chloride, 2,4,6-triisopropylbenzenesulfonyl chloride and mesitylene-2-sulfonyl chloride; sulfonyltetrazoles such as 1-toluenesulfonyltetrazole, 1-(mesitylene-2-sulfonyl)tetrazole and 1-(2,4,6-triisopropylbenzenesulfonyl)tetrazole; sulfonyltriazoles such as 3-nitro-1-toluenesulfonyl-1,2,4-triazole, 3-nitro-1-(mesitylene-2-sulfonyl)-1,2,4-triazole, and 3-nitro-1-(2,4,6-triisopropylbenzenesulfonyl)-1,2,4-triazole; and the like. 2,4,6-Triisopropylbenzenesulfonyl chloride is particularly preferred.

The cyclization reaction can be carried out in the presence of a base. Examples of the base used in the cyclization reaction include triethylamine, ethyldiisopropylamine, pyridine, lutidine, imidazole, N-methylimidazole, N-methylbenzimidazole, and the like. N-methylimidazole is particularly preferred.

The reaction temperature for the cyclization reaction can be from -10°C to the boiling point of the solvent, but it is particularly preferably from 10°C to 40°C.

The cyclization reaction can be carried out in the presence of a dehydrating agent in order to reduce the effect of moisture. The dehydrating agent used in the cyclization reaction can be any of those conventionally used in reactions, but molecular sieves are particularly preferred.

The cyclization reaction generally employs a reaction solvent. The reaction solvent is not limited as long as it does not affect the condensation, but it is preferably an aprotic solvent, and particularly preferably acetonitrile, dichloromethane or THF.

The method for synthesizing the compound represented by Formula [2] is not particularly limited, but mention may be made of a method for removing, as desired, the protective groups for B₁, B₄, R₁, R₄ and R₅ of the compound represented by Formula [6], as illustrated in the following Reaction Scheme [8]:

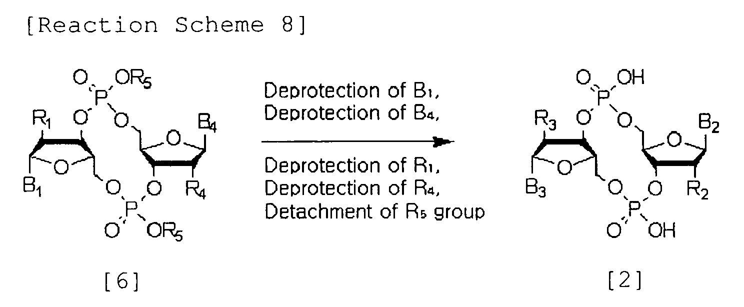

The order of removing each of the protective groups is not particularly limited, except those cases requiring particular consideration, such as one in which purification is needed in a step where some protective groups remain, or one in which the stability of the compound changes with the order of removing the protective groups. A plurality of protective groups can be simultaneously removed under the same reaction conditions, for the purpose of reducing the number of the processes.

The protective group represented by R₅ of the compound represented by Formula [6] is preferably such that the R₅ group of the compounds represented by Formulas [3], [4], [5] and [6] is stable under the reaction conditions for removing the R₆ group in Formula [5] or Formula [6], or under the reaction conditions for synthesizing the compound represented by Formula [6] by cyclizing the compound represented by Formula [5]. Furthermore, it is particularly preferable to use the protective group which can be removed under the same conditions as the reaction conditions for removing the protective group for R₁, R₄, B₁ or B₄ of the compound represented by Formula [6], as the R₅ group of the compound represented by Formula [6], for the purpose of simplifying the process of synthesizing the compound represented by Formula [2] from the compound represented by Formula [6]. For such protective group, an allyl group or a 2-cyanoethyl group is preferred.

The protective group for R₁, R₄, B₁ or B₄ of the compound represented by Formula [6] is preferably the same groups for R₁, R₄, B₁ or B₄ in the compound represented by Formula [5] .

For example, when R₅ of Formula [6] is an allyl group, deprotection of R₁, R₄, B₁ or B₄ and detachment of the R₅ group can be carried out simultaneously using the above-described zero-valent palladium, divalent palladium complex or divalent palladium salt, under the above-described reaction conditions using the palladium compounds.

The protective group for R₁, R₄, B₁ and B₄ of the compound represented by Formula [6] may be exemplified by the above-described allyl groups.

When the hydroxyl protecting group for R₁ or R₄ of the compound represented by Formula [6] is the above-described silyl group or the alkoxyalkyl group substituted with a silyl group for R₁ or R₄ of the compound represented by the Formula [5], and when the protective group of B₁ or B₄ is the above-described allyl group, deprotection of B₁ or B₄ and detachment of the R₅ group can be carried out simultaneously using the above-described zero-valent palladium, divalent palladium complexes or divalent palladium salts under the above-described reaction conditions using the palladium compounds, and then deprotection of R₁ or R₄ can be carried out using the above-described fluorides under the above-described reaction conditions using the fluorides.

Moreover, when R₅ in Formula [6] is a 2-cyanoethyl group, deprotection of R₁, R₄, B₁ or B₄ and detachment of the R₅ group can be carried out simultaneously using the above-described ammonia or amines under the above-described reaction conditions using the ammonia or amines.

The protective group of R₁ or R₄ of the compound represented by Formula [6] may be exemplified by the above-described acyl groups for R₁ or R₄ of the compound represented by Formula [5], and the protective group of B₁ and B₄ may be exemplified by the above-described acyl groups or amidines for B₁ or B₄ of the compound represented by Formula [5].

When the hydroxyl protecting group for R₁ and R₄ of the compound represented by Formula [6] is the above-described silyl group or the alkoxyalkyl substituted with a silyl group, and when the protective group of B₁ and B₄ is the above-describe acyl groups or the amidines, deprotection of B₁ or B₄ and detachment of the R₅ group can be carried out simultaneously using the above-described ammonia or amines under the above-described reaction conditions using the ammonia or amines, and then deprotection of R₁ or R₄ can be carried out using the above-described fluorides under the above-described reaction conditions using the fluorides.

When the compound represented by Formula [2] is synthesized from the compound represented by Formula [6], it is desirable to use the conditions under which the compound represented by Formula [2] is stable, for the reaction conditions under which deprotection of B₁, B₄, R₁ and R₄ of the compound represented by Formula [6] and detachment of the R₅ group are carried out.

The compound represented by Formula [1] of the invention may be exemplified by N²-(allyloxycarbonyl)-O⁶⁻allyl-2'-O-(t-butyldimethylsilyl)guanosine 3'-O-(allyl 2-cyanoethylphosphate), 2'-O-(t-butyldimethylsilyl)-N²⁻(dimethylaminomethylene)-guanosine 3'-O- (allyl 2-cyanoethylphosphate), N⁶-(allyloxycarbonyl)-2'-O-(t-butyldimethylsilyl)adenosine 3'-0-(allyl 2-cyanoethylphosphate), 2'-O-(t-butyldimethylsilyl)-N⁶⁻benzoyladenosine 3'-O-(allyl 2-cyanoethylphosphate), N⁴⁻(allyloxycarbonyl)-2'-O-(t-butyldimethylsilyl)cytidine 3'-0-(allyl 2-cyanoethylphosphate), 2'-O-(t-butyldimethylsilyl)-N⁴-benzoylcytidine 3'-O-(allyl 2-cyanoethyl phosphate), N²⁻(allyloxycarbonyl)-O⁶-allyl-2'-deoxyguanosine 3'-O-(allyl 2-cyanoethylphosphate), N²-(dimethylaminomethylene)-2'-deoxyguanosine 3'-0-(allyl 2-cyanoethylphosphate), N⁶⁻(allyloxycarbonyl)-2'-deoxyadenosine 3'-O-(allyl 2-cyanoethylphosphate), N⁶-benzoyl-2'-deoxyadenosine 3'-0-(allyl 2-cyanoethylphosphate), N⁶-(allyloxycarbonyl)-2'-deoxy-2-chloroadenosine 3'-O-(allyl 2-cyanoethylphosphate), N⁶-benzoyl-2'-deoxy-2-chloroadenosine 3'-0-(allyl 2-cyanoethylphosphate), N⁶-(allyloxycarbonyl)-2'-deoxy-2-fluoroadenosine 3'-O-(allyl 2-cyanoethylphosphate), N⁶⁻benzoyl-2'-deoxy-2-fluoroadenosine 3'-0-(allyl 2-cyanoethylphosphate), N⁴-(allyloxycarbonyl)-2'-deoxycytidine 3'-0-(allyl 2-cyanoethylphosphate), N⁴-benzoyl-2'-deoxycytidine 3'-O-(allyl 2-cyanoethylphosphate), N⁴⁻(allyloxycarbonyl)-2'-deoxy-5-fluorocytidine 3'-O-(allyl 2-cyanoethylphosphate), N⁴-benzoyl-2'-deoxy-5-fluorocytidine 3'-O-(allyl 2-cyanoethylphosphate), N⁴-(allyloxycarbonyl)-2'-deoxy-5-azacytidine 3'-O-(allyl 2-cyanoethylphosphate), N⁴-benzoyl-2'-deoxy-5-azacytidine 3'-O-(allyl 2-cyanoethylphosphate), N⁴-(allyloxycarbonyl)-2'-deoxy-5-fluorouridine 3'-O-(allyl 2-cyanoethylphosphate), N⁴― benzoyl-2'-deoxy-5-fluorouridine 3'-O-(allyl 2-cyanoethylphosphate), N²-(allyloxycarbonyl)-O⁶-allyl-2'-deoxy-2'-fluoroguanosine 3'-O-(allyl 2-cyanoethylphosphate), N²-(dimethylaminomethylene)-2'-deoxy-2'-fluoroguanosine 3'-O-(allyl 2-cyanoethylphosphate), N⁶-(allyloxycarbonyl)-2'-deoxy-2'-fluoroadenosine 3'-O-(allyl 2-cyanoethylphosphate), N⁶-benzoyl-2'-deoxy-2'-fluoroadenosine 3'-O-(allyl 2-cyanoethylphosphate), N⁴-(allyloxycarbonyl)-2'-deoxy-2'-fluorocytidine 3'-O-(allyl 2-cyanoethylphosphate), N⁴⁻benzoyl-2'-deoxy-2'-fluorocytidine 3'-O-(allyl 2-cyanoethylphosphate) or the like.

The compound represented by Formula [5] of the invention may be exemplified by allyl [N²⁻(allyloxycarbonyl)-O⁶-allyl-2'-O-(t-butyldimethylsilyl)guanylyl](3'→5')[N²-(allyloxycarbonyl)-O⁶-allyl-2'-O-(t-butyldimethylsilyl)guanosine 3'-O-(allyl 2-cyanoethylphosphate)], allyl [N⁶-(allyloxycarbonyl)-2'-O-(t-butyldimethylsilyl)adenylyl](3'→5')[N²-(allyloxycarbonyl)-O⁶-allyl-2'-O-(t-butyldimethylsilyl)guanosine 3'-O-(allyl 2-cyanoethylphosphate)], allyl [N²-(allyloxycarbonyl)-O⁶-allyl-2'-O-(t-butyldimethylsilyl)guanylyl](3'→5')[N⁴⁻(allyloxycarbonyl)-2'-O-(t-butyldimethylsilyl)cytidine 3'-O-(allyl 2-cyanoethylphosphate)], allyl [N²⁻(allyloxycarbonyl)-O⁶-allyl-2'-O-(t-butyldimethylsilyl)guanylyl](3'→5')[2'-0-(t-butyldimethylsilyl)uridine 3'-O-(allyl 2-cyanoethylphosphate)], allyl [N⁶-(allyloxycarbonyl)-2'-O-(t-butyldimethylsilyl)adenylyl](3'→5')[N²-(allyloxycarbonyl)-O⁶-allyl-2'-O-(t-butyldimethylsilyl)guanosine 3'-O-(allyl 2-cyanoethylphosphate)], allyl [N⁶-(allyloxycarbonyl)-2'-O-(t-butyldimethylsilyl)adenylyl](3'→5')[N⁶-(allyloxycarbonyl)-2'-O-(t-butyldimethylsilyl)adenosine 3'-O-(allyl 2-cyanoethylphosphate)], allyl [N⁶-(allyloxycarbonyl)-2'-O-(t-butyldimethylsilyl)adenylyl](3'→5')[N⁴-(allyloxycarbonyl)-2'-O-(t-butyldimethylsilyl)cytidine 3'-O-(allyl 2-cyanoethylphosphate)], 2-cyanoethyl [2'-O-(t-butyldimethylsilyl)-N²⁻(dimethylaminomethylene)guanylyl](3'→5')[2'-O-(t-butyldimethylsilyl)-N²-(dimethylaminomethylene)guanosine 3'-O-(allyl 2-cyanoethylphosphate)], 2-cyanoethyl [2'-O-(t-butyldimethylsilyl)-N²⁻(dimethylaminomethylene)guanylyl](3'→5')[2'-O-(t-butyldimethylsilyl)-N⁶-benzoyladenosine 3'-O-(allyl 2-cyanoethylphosphate)], 2-cyanoethyl [2'-O-(t-butyldimethylsilyl)-N²⁻(dimethylaminomethylene)guanylyl](3'→5')[2'-O-(t-butyldimethylsilyl)-N⁴-benzoylcytidine 3'-O-(allyl 2-cyanoethylphosphate)], 2-cyanoethyl [2'-O-(t-butyldimethylsilyl)-N⁴-acetylcytidine](3'→5')[2'-O-(t-butyldimethylsilyl)-N²-(dimethylaminomethylene)guanosine 3'-O-(allyl 2-cyanoethylphosphate)], 2-cyanoethyl [2'-O-(t-butyldimethylsilyl)-N⁶-benzoyladenosine](3'→5')[2'-O-(t-butyldimethylsilyl)-N⁶-benzoyladenosine 3'-O-(allyl 2-cyanoethylphosphate)], 2-cyanoethyl [2'-O-(t-butyldimethylsilyl)-N⁴-benzoylcytidine](3'→5')[2'-O-(t-butyldimethylsilyl)-N⁴-benzoylcytidine 3'-O-(allyl 2-cyanoethylphosphate)], allyl [N²-(allyloxycarbonyl)-O⁶-allyl-2'-deoxyguanylyl](3'→5')[N²-(allyloxycarbonyl)-O⁶-allyl-2'-O-deoxyguanosine 3'-O-(allyl 2-cyanoethylphosphate)], allyl [N⁶-(allyloxycarbonyl)-2'-deoxyadenylyl](3'→5')[N²⁻(allyloxycarbonyl)-O⁶-allyl-2'-deoxyguanosine 3'-O-(allyl 2-cyanoethylphosphate)], allyl [N²-(allyloxycarbonyl)-O⁶-allyl-2'-deoxyguanylyl](3'→5')[N⁴-(allyloxycarbonyl)-2'-deoxycytidine 3'-O-(allyl 2-cyanoethylphosphate)], allyl [N²-(allyloxycarbonyl)-O⁶-allyl-2'-deoxyguanylyl](3'→5')[2'-deoxyuridine 3'-O-(allyl 2-cyanoethylphosphate)], allyl [N⁶⁻(allyloxycarbonyl)-2'-deoxyadenylyl](3'→5')[N²⁻(allyloxycarbonyl)-O⁶-allyl-2'-deoxyguanosine 3'-O-(allyl 2-cyanoethylphosphate)], allyl [N⁶-(allyloxycarbonyl)-2'-deoxyadenylyl](3'→5')[N⁶-(allyloxycarbonyl)-2'-deoxyadenosine 3'-O-(allyl 2-cyanoethylphosphate)], allyl [N⁶-(allyloxycarbonyl)-2'-deoxy-2-chloroadenylyl](3'→5')[N⁶⁻(allyloxycarbonyl)-2'-deoxy-2-chloroadenosine 3'-O-(allyl 2-cyanoethylphosphate)], allyl [N⁶-(allyloxycarbonyl)-2'-deoxy-2-fluoroadenylyl](3'→5')[N⁶-(allyloxycarbonyl)-2'-deoxy-2-fluoroadenosine 3'-O-(allyl 2-cyanoethylphosphate)], allyl [N⁶-(allyloxycarbonyl)-2'-deoxyadenylyl] (3'→5') [N⁴⁻(allyloxycarbonyl)-2'-deoxycytidine 3'-O-(allyl 2-cyanoethylphosphate)], 2-cyanoethyl [2'-deoxy-N²⁻(dimethylaminomethylene)guanylyl](3'→5')[2'-deoxy-N²⁻(dimethylaminomethylene)guanosine 3'-O-(allyl 2-cyanoethylphosphate)], 2-cyanoethyl [2'-deoxy-N²⁻(dimethylaminomethylene)guanylyl](3'→5')[2'-deoxy-N⁶⁻benzoyladenosine 3'-O-(allyl 2-cyanoethylphosphate)], 2-cyanoethyl [2'-deoxy-N²⁻(dimethylaminomethylene)guanylyl](3'→5')[2'-deoxy-N⁴⁻benzoylcytidine 3'-O-(allyl 2-cyanoethylphosphate)], 2-cyanoethyl [2'-deoxy-N⁴-acetylcytidine](3'→5')[2'-deoxy-N²⁻(dimethylaminomethylene)guanosine 3'-O-(allyl 2-cyanoethylphosphate)], 2-cyanoethyl [2'-deoxy-N⁶⁻benzoyladenosine](3'→5')[2'-deoxy-N6-benzoyladenosine 3'-O-(allyl 2-cyanoethylphosphate)], 2-cyanoethyl [2'-deoxy-N⁴⁻benzoylcytidine](3'→5')[2'-deoxy-N4-benzoylcytidine 3'-O-(allyl 2-cyanoethylphosphate)], allyl [N²⁻(allyloxycarbonyl)-O⁶-allyl-2'-deoxy-2'-fluoroguanylyl](3'→5')[N²-(allyloxycarbonyl)-O⁶-allyl-2'-O-deoxy-2'-fluoroguanosine 3'-O-(allyl 2-cyanoethylphosphate)], allyl [N⁶-(allyloxycarbonyl)-2'-deoxy-2'-fluoroadenylyl] (3'→5') [N²-(allyloxycarbonyl)-O⁶-allyl-2'-deoxy-2'-fluoroguanosine 3'-O-(allyl 2-cyanoethylphosphate)], allyl [N²-(allyloxycarbonyl)-O⁶-allyl-2'-deoxy-2'-fluoroguanylyl](3'→5')(N⁴-(allyloxycarbonyl)-2'-deoxy-2'-fluorocytidine 3'-O-(allyl 2-cyanoethylphosphate)], allyl [N²-(allyloxycarbonyl)-O⁶-allyl-2'-deoxy-2'-fluoroguanylyl](3'→5')[2'-deoxy-2'-fluorouridine-3'-O-(allyl 2-cyanoethylphosphate)], allyl [N⁶⁻(allyloxycarbonyl)-2'-deoxy-2'-fluoroadenylyl](3'→5')[NZ-(allyloxycarbonyl)-O⁶-allyl-2'-deoxy-2'-fluoroguanosine 3'-O-(allyl 2-cyanoethylphosphate)], allyl [N⁶⁻(allyloxycarbonyl)-2'-deoxy-2'-fluoroadenylyl](3'→5')[N⁶⁻(allyloxycarbonyl)-2'-deoxy-2'-fluoroadenosine 3'-O-(allyl 2-cyanoethylphosphate)], allyl [N⁶-(allyloxycarbonyl)-2'-deoxy-2'-fluoroadenylyl](3'→5') [N⁴-(allyloxycarbonyl)-2'-deoxy-2'-fluorocytidine 3'-O-(allyl 2-cyanoethylphosphate)], 2-cyanoethyl [2'-deoxy-N²-(dimethylaminomethylene)-2'-fluoroguanylyl](3'→5')[2'-deoxy-N²-(dimethylaminomethylene)-2'-fluoroguanosine 3'-O-(allyl 2-cyanoethylphosphate)], 2-cyanoethyl [2'-deoxy-N²-(dimethylaminomethylene)-2'-fluoroguanylyl](3'→5')[2'-deoxy-N⁶-benzoyl-2'-fluoroadenosine 3'-O-(allyl 2-cyanoethylphosphate)], 2-cyanoethyl [2'-deoxy-N²-(dimethylaminomethylene)-2'-fluoroguanylyl](3'→5')[2'-deoxy-N⁴-benzoyl-2'-fluorocytidine 3'-O-(allyl 2-cyanoethylphosphate)], 2-cyanoethyl [2'-deoxy-N⁴-acetyl-2'-fluorocytidine](3'→5')[2'-deoxy-N²⁻(dimethylaminomethylene)-2'-fluoroguanosine 3'-O-(allyl 2-cyanoethylphosphate)], 2-cyanoethyl [2'-deoxy-N⁶-benzoyl-2'-fluoroadenosine](3'→5')[2'-deoxy-N⁶-benzoyl-2'-fluoroadenosine 3'-O-(allyl 2-cyanoethylphosphate)], 2-cyanoethyl [2'-deoxy-N⁴-benzoyl-2'-fluorocytidine](3'→5')[2'-deoxy-N⁴-benzoyl-2'-fluorocytidine 3'-O-(allyl 2-cyanoethylphosphate)], 2-cyanoethyl [2'-deoxy-N⁴-benzoyl-5-fluorocytidine](3'→5')[2'-deoxy-N⁴-benzoyl-5-fluorocytidine 3'-O-(allyl 2-cyanoethylphosphate)], 2-cyanoethyl [2'-deoxy-N⁴-benzoyl-5-azacytidine](3'→5')[2'-deoxy-N⁴-benzoyl-5-azacytidine 3'-O-(allyl 2-cyanoethylphosphate)], 2-cyanoethyl [2'-deoxy-5-fluorouridine](3'→5')[2'-deoxy-5-fluorouridine 3'-0-(allyl 2-cyanoethylphosphate)], 2-cyanoethyl [2'-deoxy-5-trifluoromethyluridine](3'→5')[2'-deoxy-5-trifluoromethyluridine 3'-O-(allyl 2-cyanoethylphosphate)] or the like.

The compound represented by Formula [6] of the invention may be exemplified by cyclic bis (3'→5') bis[N²⁻(allyloxycarbonyl)-O⁶-allyl-2'-O-(t-butyldimethylsilyl)guanylic acid] diallyl ester, cyclic bis(3'→5')bis[N⁶-(allyloxycarbonyl)-2'-O-(t-butyldimethylsilyl)adenylic acid] diallyl ester, cyclic bis(3'→5')bis[N⁴-(allyloxycarbonyl)-2'-O-(t-butyldimethylsilyl)cytidylic acid] diallyl ester, cyclic bis(3'→5')bis[2'-O-(t-butyldimethylsilyl)-N²⁻(dimethylaminomethylene)guanylic acid] bis(2-cyanoethyl) ester, cyclic bis(3'→5')bis[2'-O-(t-butyldimethylsilyl)-N⁴⁻acetylcytidylic acid] bis(2-cyanoethyl) ester, cyclic bis(3'→5')bis[2'-O-(t-butyldimethylsilyl)-N⁴⁻benzoylcytidylic acid] bis(2-cyanoethyl) ester, cyclic bis(3'→5')bis[2'-O-(t-butyldimethylsilyl)-N⁶-benzoyladenylic acid] bis(2-cyanoethyl) ester, cyclic bis(3'→5')[N⁶⁻(allyloxycarbonyl)-2'-O-(t-butyldimethylsilyl)adenyl][N²⁻(allyloxycarbonyl)-O⁶-allyl-2'-0-(t-butyldimethylsilyl)guanylic acid] diallyl ester, cyclic bis(3'→5')bis[N⁶-(allyloxycarbonyl)-2'-O-(t-butyldimethylsilyl)adenylic acid] diallyl ester, cyclic bis (3'→5' ) [N⁶- (allyloxycarbonyl)-2'-O-(t-butyldimethylsilyl)adenylyl] [N⁴-(allyloxycarbonyl)-2'-O-(t-butyldimethylsilyl)cytidylic acid] diallyl ester, cyclic bis(3'→5')[2'-O-(t-butyldimethylsilyl)-N⁴-acetylcytidylyl] [2'-0-(t-butyldimethylsilyl)-N²⁻(dimethylaminomethylene)guanylic acid] bis(2-cyanoethyl) ester, cyclic bis(3'→5')[2'-0-(t-butyldimethylsilyl)-N²⁻(dimethylaminomethylene)guanylyl] [2'-O-(t-butyldimethylsilyl)-N⁴-acetylcytidylic acid] bis(2-cyanoethyl) ester, cyclic bis(3'→5')[2'-O-(t-butyldimethylsilyl)uridylyl] [2'-0-(t-butyldimethylsilyl)-N⁴-benzoylcytidylic acid] bis(2-cyanoethyl) ester, cyclic bis (3'→5') [2'-O- (t-butyldimethylsilyl) -N²⁻(dimethylaminomethylene)guanylyl] [2'-O-(t-butyldimethylsilyl)-N⁶-benzoyladenylic acid] bis(2-cyanoethyl) ester, cyclic bis(3'→5')bis[N²⁻(allyloxycarbonyl)-O⁶ allyl-2'-deoxyguanylic acid] diallyl ester, cyclic bis(3'→5')bis[N⁶-(allyloxycarbonyl)-2'-deoxyadenylic acid] diallyl ester, cyclic bis(3'→5') bis[N⁴⁻(allyloxycarbonyl)-2'-deoxycytidylic acid] diallyl ester, cyclic bis(3'→5')bis[2'-deoxy-N²⁻(dimethylaminomethylene)guanylic acid] bis(2-cyanoethyl) ester, cyclic bis(3'→5')bis[2'-deoxy-N⁴-acetylcytidylic acid] bis(2-cyanoethyl) ester, cyclic bis (3'→5') bis [2'-deoxy-N⁴-benzoylcytidylic acid] bis(2-cyanoethyl) ester, cyclic bis(3'→5')bis[2'-deoxy-N⁶-benzoyladenylic acid] bis(2-cyanoethyl) ester, cyclic bis(3'→5')bis[2'-deoxy-N⁶⁻benzoyl-2-chloroadenylic acid] bis(2-cyanoethyl) ester, cyclic bis(3'→5')bis[2'-deoxy-N⁶-benzoyl-2-fluoroadenylic acid] bis (2-cyanoethyl) ester, cyclic bis (3'→5')[N⁶⁻(allyloxycarbonyl) -2' -deoxyadenyl] [N²- (allyloxycarbonyl) -O⁶⁻allyl-2'-deoxyguanylic acid] diallyl ester, cyclic bis(3'→5')bis[N⁶-(allyloxycarbonyl)-2'-deoxyadenylic acid] diallyl ester, cyclic bis(3'→5') [N⁶-(allyloxycarbonyl)-2'-deoxyadenylyl][N⁴-(allyloxycarbonyl)-2'-deoxycytidylic acid] diallyl ester, cyclic bis(3'→5')[2'-deoxy-N⁴⁻acetylcytidylyl][2'-deoxy-N²⁻(dimethylaminomethylene)guanylic acid] bis(2-cyanoethyl) ester, cyclic bis(3'→5')[2'-deoxy-N²⁻(dimethylaminomethylene)guanylyl][2'-deoxy-N⁴⁻acetylcytidylic acid] bis(2-cyanoethyl) ester, cyclic bis(3'→5')[2-deoxyuridylyl][2-deoxy-N⁴-benzoylcytidylic acid] bis (2-cyanoethyl) ester, cyclic bis(3'→5')[2'-deoxy-N²-(dimethylaminomethylene)guanylyl][2'-deoxy-N⁶⁻benzoyladenylic acid] bis(2-cyanoethyl) ester, cyclic bis(3'→5')bis[N²-(allyloxycarbonyl)-O⁶-allyl-2'-deoxy-2'-fluoroguanylic acid] diallyl ester, cyclic bis(3'→5')bis[N⁶⁻(allyloxycarbonyl)-2'-deoxy-2'-fluoroadenylic acid] diallyl ester, cyclic bis(3→5')bis[N⁴-(allyloxycarbonyl)-2'-deoxy-2'-fluorocytidylic acid] diallyl ester, cyclic bis(3'→5')bis[2'-deoxy-N²-(dimethylaminomethylene)-2'-fluoroguanylic acid] bis(2-cyanoethyl) ester, cyclic bis(3'→5')bis[2'-deoxy-N⁴-acetyl-2'-fluorocytidylic acid] bis(2-cyanoethyl) ester, cyclic bis(3'→5')bis[2'-deoxy-N⁴⁻benzoyl-2'-fluorocytidylic acid] bis(2-cyanoethyl) ester, cyclic bis(3'→5')bis[2'-deoxy-N⁴-acetyl-5-fluorocytidylic acid] bis(2-cyanoethyl) ester, cyclic bis(3'→5')bis[2'-deoxy-N⁴-benzoyl-5-fluorocytidylic acid] bis(2-cyanoethyl) ester, cyclic bis(3'→5')bis[2'-deoxy-N⁴-acetyl-5-azacytidylic acid] bis(2-cyanoethyl) ester, cyclic bis(3'→5')bis[2'-deoxy-N⁴-benzoyl-5-azacytidylic acid] bis (2-cyanoethyl) ester, cyclic bis (3'→5') bis [2'-deoxy-5-fluorouridylic acid] bis(2-cyanoethyl) ester, cyclic bis(3'→5')bis[2'-deoxy-5-trifluoromethyluridylic acid] bis(2-cyanoethyl) ester, cyclic bis(3'→5')bis[2'-deoxy-N⁶⁻benzoyl-2'-fluoroadenylic acid] bis(2-cyanoethyl) ester, cyclic bis (3'→5') [N⁶-(allyloxycarbonyl)-2'-deoxy-2'-fluoroadenyl][N²-(allyloxycarbonyl)-O⁶-allyl-2'-deoxy-2'-fluoroguanylic acid] diallyl ester, cyclic bis(3'→5')bis[N⁶⁻(allyloxycarbonyl)-2'-deoxy-2'-fluoroadenylic acid] diallyl ester, cyclic bis (3'→5') [N⁶-(allyloxycarbonyl)-2'-deoxy-2'-fluoroadenylyl][N⁴-(allyloxycarbonyl)-2'-deoxy-2'-fluorocytidylic acid] diallyl ester, cyclic bis(3'→5')[2'-deoxy-N⁴-acetyl-2'-fluorocytidylyl][2'-deoxy-N²⁻(dimethylaminomethylene)-2'-fluoroguanylic acid] bis(2-cyanoethyl) ester, cyclic bis(3'→5')[2'-deoxy-N²⁻(dimethylaminomethylene)-2'-fluoroguanylyl][2'-deoxy-N⁴⁻acetyl-2'-fluorocytidylic acid] bis(2-cyanoethyl) ester, cyclic bis(3'→5')[2'-deoxy-2'-fluorouridylyl][2'-deoxy-N⁴⁻benzoyl-2'-fluorocytidylic acid] bis(2-cyanoethyl) ester, cyclic bis(3'→5')[2'-deoxy-N²-(dimethylaminomethylene)-2'-fluoroguanylyl][2'-deoxy-N⁶-benzoyl-2'-fluoroadenylic acid] bis(2-cyanoethyl) ester or the like.

The compound represented by Formula [2], the compound represented by Formula [5], and the compound represented by Formula [6] described above can be separated and recovered from the reaction mixtures obtained from the synthetic processes for the respective compounds, by a known method such as, for example, column chromatography or crystallization.

Hereinafter, the invention will be described in more detail with reference to Examples, but the invention is not intended to be limited by these Examples.

### EXAMPLE 1

### Synthesis of N²-(allyloxycarbonyl)-O⁶-allyl-2'-0-(t-butyldimethylsilyl)guanosine 3'-O-(allyl 2-cyanoethylphosphate) (Compound [8])

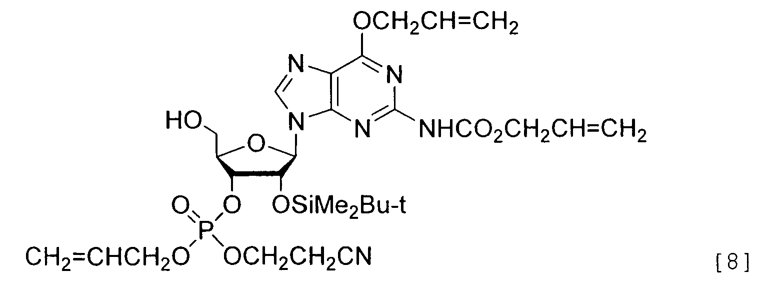

To a solution of 2.0 g (2.0 mmol) of N²⁻(allyloxycarbonyl)-O⁶-allyl-2'-O-(t-butyldimethylsilyl)-5'-0-(4,4'-dimethoxytrityl)guanosine 3'-0-(allyl N,N-diisopropylphosphoroamidite) (synthesized by the method described in Org. Lett., 3, p. 815 (2001)) and 200 mg of Molecular Sieves 3A in dry acetonitrile, 0.16 mL (2.4 mmol) of 2-cyanoethanol was added and the mixture was stirred at room temperature for 30 minutes, then 0.67 g (4.0 mmol) of imidazolium perchlorate was added, and the mixture was stirred for another 30 minutes. A 1.0 M solution of 2-butanone peroxide in 4 mL of dimethyl phthalate/toluene was further added thereto, and the mixture was stirred for 5 minutes. After removing the Molecular Sieves 3A by filtration, ethyl acetate was added to the mixture, and the mixture was washed with a saturated aqueous sodium bicarbonate solution and saturated brine, dried and then concentrated under reduced pressure. The residue was dissolved in 20 mL of dichloromethane, 3.3 mL (40 mmol) of dichloroacetic acid was added dropwise to the solution under stirring with ice-cooling, and the mixture was stirred for 5 minutes. The reaction solution was added dropwise to 100 mL of a saturated aqueous sodium bicarbonate solution, the organic layer was separated, and then the aqueous layer was extracted with dichloromethane. The organic layer was combined, and the mixture was dried over sodium sulfate and then concentrated under reduced pressure. The concentrated residue was purified by silica gel chromatography (40 g of silica gel, hexane:ethyl acetate (1:1) to hexane:ethyl acetate:methanol (10:10:1)) to yield 1.38 g of the title compound as a colorless amorphous material. Yield: 95%. IR(CH₂Cl₂) : cm⁻¹ 3420, 3048, 2305, 1757, 1607, 1524, 1462, 1412, 1294, 1190, 1038, 756; ¹H NMR (CDCl₃) δ -0.27 (s, 3H), -0.03 (s, 3H), 0.74 (s, 9H), 2.94 (t, J= 6.0 Hz, 2H), 3.85-3.97 (m, 2H), 4.30-4.43 (m, 3H), 4.70 (m, 4H), 5.00 (m, 1H), 5.08-5.10 (m, 2H), 5.24-5.52 (m, 7H), 6.00-6.20 (m, 4H), 8.39 (s, 1H); ³¹P NMR (CD₃OD) δ -4.69, -4.54.

### EXAMPLE 2

### Synthesis of allyl [N²-(allyloxycarbonyl)-O⁶-allyl-2'-0-(t-butyldimethylsilyl)guanylyl] (3'→5') [N²⁻(allyloxycarbonyl)-O⁶-allyl-2'-O-(t-butyldimethylsilyl)guanosine 3'-0-(allyl 2-cyanoethylphosphate)] (Compound [9])

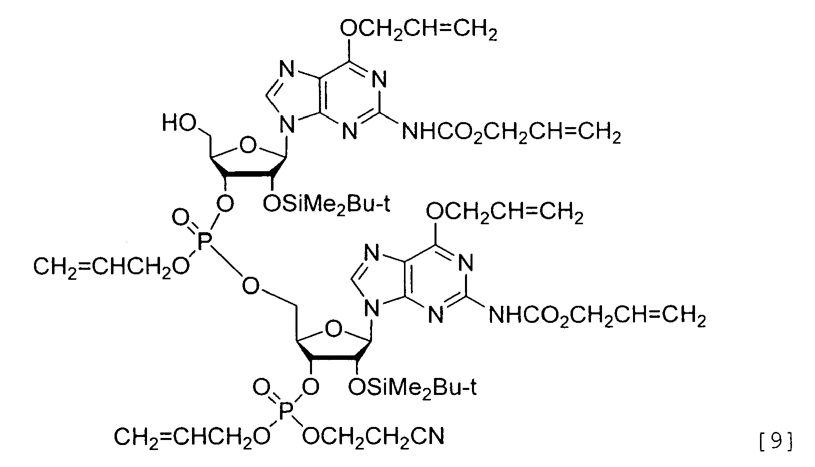

A solution of 1.6 g (1.6 mmol) of N²-(allyloxycarbonyl)-O⁶-allyl-2'-O-(t-butyldimethylsilyl)-5'-O-(4,4'-dimethoxytrityl)guanosine 3'-O-(allyl N,N-diisopropylphosphoroamidite), 1.1 g (1.6 mmol) of Compound [8] and 0.2 g of Molecular Sieves 3A in 15 mL of dry acetonitrile was stirred at room temperature for 30 minutes, and then the mixture was mixed with 0.54 g (3.2 mmol) of imidazolium perchlorate and stirred for another 30 minutes. A 1.0 M solution of 2-butanone peroxide in 3.2 mL of dimethyl phthalate/toluene was further added to the mixture, and the mixture was stirred for 5 minutes. After removing the Molecular Sieves 3A by filtration, the mixture was concentrated under reduced pressure. The residue was dissolved in 20 mL of dichloromethane, 3.3 mL (40 mmol) of dichloroacetic acid was added dropwise to the solution under stirring with ice-cooling, and the mixture was stirred for 5 minutes. The reaction solution was added dropwise to 100 mL of a saturated aqueous sodium bicarbonate solution, the organic layer was separated, and then the aqueous layer was extracted with dichloromethane. The organic layer was combined, and the mixture was dried over sodium sulfate and then concentrated under reduced pressure. The concentrated residue was purified by silica gel chromatography (silica gel: 40 g, hexane:ethyl acetate (1:1) to hexane:ethyl acetate:methanol (20:20:1)) to yield 1.95 g of the title compound (mixture of diastereomers) as a colorless amorphous material. Yield: 94%.
¹H NMR (CDCl₃) δ -0.36-0.03 (m, 12H), -0.68-0.77 (m, 18H), 1.78 (br s, 1H), 2.79-2.81 (m, 2H), 3.79-3.96 (m, 4H), 4.30-4.36 (m, 4H), 4.48-4.71 (m, 14H), 4.99-5.50 (m, 14H), 5.75-6.19 (m, 8H), 7.26-8.67 (m, 4H); ³¹P NMR (CD₃OD) δ -1.32, - 1.24, -1.11, -1.05, -0.88, -0.81; HRMS (MALDI⁺) calcd for C₅₅H₈₃N₁₁O₁₉P₂Si₂⁺ (M+H⁺) 1318.4797, found 1318.5267.

### EXAMPLE 3

### Synthesis of cyclic bis(3'→5')bis[N²⁻(allyloxycarbonyl)-O⁶-allyl-2'-O-(t-butyldimethylsilyl)guanylic acid] diallyl ester (Compound [10]: mixture of diastereomers [10a] and [10b])

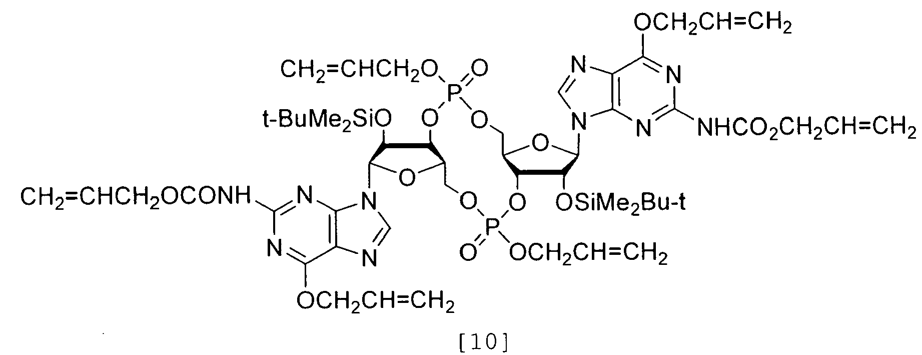

To a solution of 0.66 g (0.5 mmol) of Compound [9] in 10 mL of methanol, 1 mL of a 28% aqueous ammonia solution was added dropwise, and the mixture was stirred at room temperature for 30 minutes. After concentration under reduced pressure, 20 mL of toluene was further added, and the mixture was concentrated under reduced pressure (3 times). The residue was dissolved in 100 mL of THF, and Molecular Sieves 4A was added thereto to dehydrate the solution. Then, 0.08 mL (1.0 mmol) of N-methylimidazole and 0.3 g (1.0 mmol) of triisopropylbenzenesulfonyl chloride were added thereto, and the mixture was stirred at room temperature for 20 hours. The residue obtained by concentrating the reaction solution under reduced pressure, was purified by silica gel chromatography (silica gel 40 g, hexane:ethyl acetate (2:1) to hexane:ethyl acetate:methanol (30:30:1)) to obtain 0.47 g of the title compound (270 mg of the diastereomer [10a] and 200 mg of the diastereomer [10b]) as a colorless amorphous material. Yield: 75%.
[10a] ¹H NMR (CDCl₃) δ -0.31, -0.21, -0.03, 0.04 (4s's, 12H), 0.76 (s, 18H), 4.03-4.09 (m, 2H), 4.26-4.34 (m, 2H), 4.54-4.69 (m, 8H), 4.74-5.86 (m, 4H), 4.97-5.15 (m, 6H), 5.21-5.52 (m, 12H), 5.64-5.68 (m, 1H), 5.74-5.82 (m, 3H), 5.94-6.01 (m, 4H), 6.08-6.18 (m, 2H), 7.53 (s, 1H), 7.78 (s, 1H), 7.81 (s, 1H), 8.03 (s, 1H); ³¹P NMR (CDCl₃) δ -2.31, 1.91; HRMS (ESI⁺) calcd for C₅₂H₇₇N₁₀O₁₈P₂Si₂⁺ (M+H⁺) 1247.4426, found 1247.4496.
[10b] ¹H NMR (CDCl₃) δ -0.22, -0.08 (2s's, 12H), 0.74 (s, 18H), 4.07 (m, 2H), 4.48-4.51 (m, 2H), 4.65-4.68 (m, 8H), 4.96 (q, J= 11 Hz, 2H), 5.03-5.13 (m, 4H), 5.19-5.58 (m, 16H), 5.77 (d, J= 7.0 Hz, 1H), 5.93-5.97 (m, 4H), 6.11-6.17 (m, 2H), 7.78 (s, 2H), 7.85 (s, 2H); ³¹P NMR (CDCl₃) δ 1.50; HRMS (ESI⁺) calcd for C₅₂H₇₇N₁₀O₁₈P₂Si₂⁺ (M+H⁺) 1247.4426, found 1247.4435.

### EXAMPLE 4

### Synthesis of cyclic bis(3'→5')diguanylic acid (Compound [11])

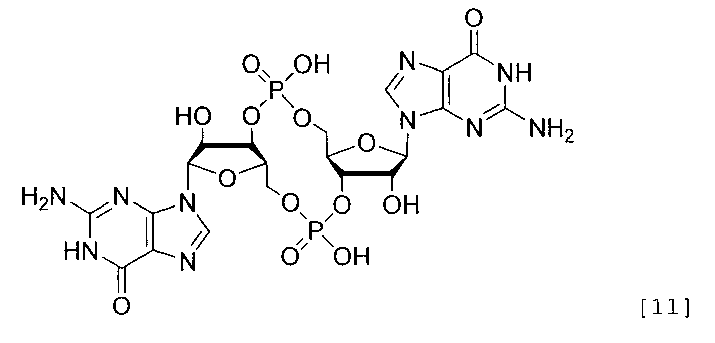

To a solution of 50 mg (0.04 mmol) of Compound [10] in 1.6 mL of THF, 16 mg (0.060 mmol) of triphenylphosphine, 0.049 mL (0.48 mmol) of n-butylamine, 0.018 mL (0.48 mmol) of formic acid, and 12 mg (0.012 mmol) of Pd₂[(C₆H₅CH=CH)₂CO]₃·CHCl₃ were added, and the mixture was stirred at room temperature for 10 minutes. 10 mL of ethyl acetate was added thereto, and the precipitate obtained was recovered by filtration and dried under reduced pressure. To the obtained white powder, 0.3 mL of a triethylamine/3HF complex was added, and the mixture was stirred at room temperature for 12 hours. 30 µL of the reaction solution was dissolved in 40 µL of a 0.1 M aqueous solution of Na₂HPO₄ and 500 µL of deuterated water, and ³¹P NMR of the solution was measured. The reaction yield was 77%. After the measurement, to the reaction solution, 1 mL of a 1 mM aqueous solution of ammonium acetate was added, and the mixture was stirred at a temperature of 30 to 40°C. The precipitate was centrifuged and purified by HPLC to obtain 12 mg of the title compound. Yield: 40%.
[HPLC conditions]
Column: COSMOSIL 5C₁₈-AR-300 (25φ x 200 mm)
Eluent: Liquid A, 1 mM aqueous solution of ammonium acetate; Liquid B, 0.2 mM solution of ammonium acetate /water-acetonitrile (20:80)

| Gradient conditions: | | | | |
|---|---|---|---|---|
| Time (min) | 0 | 8 | 55 | 63 |
| B% | 0 | 0 | 60 | 100 |

Detection wavelength: 254 nm, Flow rate: 10 mL/min UV (50 mM NH₄OAc) λ 254 nm (ε23,700); ¹H NMR (D₂O) δ 4.04-4.06 (m, 2H), 4.38-4.44 (m, 4H), 5.08 (s, 2H), 5.33 (s, 2H), 6.12 (s, 2H), 8.25 (s, 2H); ¹³C NMR (D₂O) δ 62.8, 70.9, 73.7, 80.8, 90.6, 116.4, 136.9 (weak), 150. 4 (weak), 154.1, 157.8; ³¹P NMR (D₂O) δ -0.86; HRMS (ESI⁻) calcd for C₂₀H₂₃N₁₀O₁₈P₂⁻ (M-H⁻) 689.0876, found 689.0853.

### Reference Example 1

### Synthesis of 2'-O-(t-butyldimethylsilyl)-N²⁻(dimethylaminomethylene)-3',5'-O-(di-t-butylsilyl)guanosine (Compound [12])

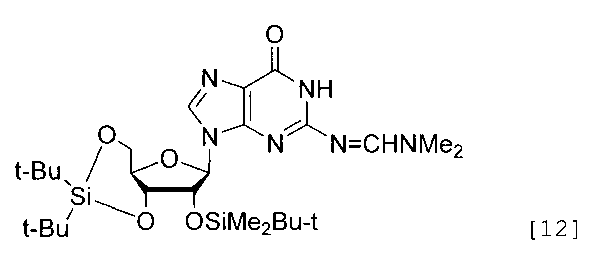

To a solution of 14.3 g (25 mmol) of 2'-O-(t-butyldimethylsilyl)-3',5'-O-(di-t-butylsilyl)guanosine (synthesized by the method described in Tetrahedron Lett., 43, p. 1983 (2002)) in 150 mL of methanol, 11.9 g (13.3 mL, 100 mol) of N,N-dimethylformamide dimethyl ether was added, and the mixture was stirred at 50°C for 5 hours. After cooling, a precipitate was recovered by filtration, washed with cold methanol, and dried under reduced pressure to obtain 14.5 g of the title compound as a white powder. Yield: 98%.
¹H NMR (CDCl₃) δ 0.14 (s, 6H), 0.93 (s, 9H), 1.05 (s, 9H), 1.07 (s, 9H), 3.13 (s, 3H), 3.34 (s, 3H), 4.00-4.06 (m, 1H), 4.18-4.21 (m, 2H), 4.42 (d, J= 3.6 Hz, 1H), 4.49-4.52 (m, 1H), 5.93 (s, 1H), 7.60 (s, 1h), 8.59 (s, 1H).

### Reference Example 2

### Synthesis of 2'-O-(t-butyldimethylsilyl)-N²⁻(dimethylaminomethylene)-5'-O-(4,4'-dimethoxytrityl)guanosine (Compound [13])

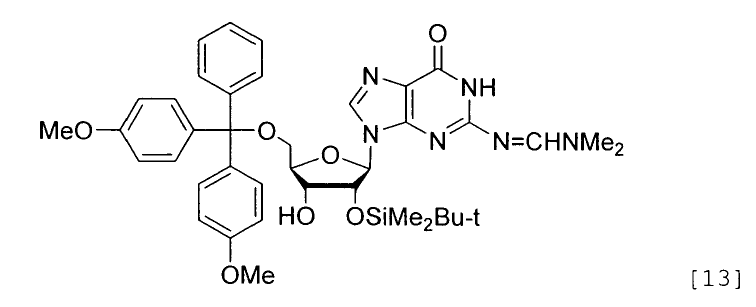

To a solution of 14.8 g (25 mmol) of 2'-0-(t-butyldimethylsilyl)-N²-(dimethylaminomethylene)-3',5'-O-(dit-butylsilyl)guanosine (Compound [12]) in 100 mL of dichloromethane, a solution of 2.0 mL (100 mmol) of cold HF-pyridine in 12 mL of pyridine was added under ice-cooling, and the mixture was stirred for 2 hours. The reaction solution was washed with water and a saturated aqueous sodium bicarbonate solution, and then concentrated under reduced pressure. The residue was dissolved in 50 mL of pyridine, and 9.3 g (27.5 mmol) of 4,4'-dimethoxytriyl chloride was added thereto. The mixture was stirred for another 12 hours. 5 mL of methanol was added, and the mixture was concentrated. The residue was dissolved in ethyl acetate and washed with water, a saturated aqueous sodium bicarbonate solution and saturated brine. The organic layer was purified by silica gel chromatography (silica gel 400 g) to obtain 16 g of the title compound as a colorless amorphous material. Yield: 86%.
¹H NMR (CDCl₃) δ -0.13 (s, 3H), 0.01 (s, 3H), 0.83 (s, 9H), 2.80 (d, J= 3.7 Hz, 2H), 3.02 (s, 3H), 3.07 (s, 3H), 3.35 (dd, J= 4.0, 10.5 Hz, 2H), 3.77 (s, 6H), 4.20 (q, J= 3.5 Hz, 1H), 4 . 30 (q, J= 3.5 Hz, 1H), 4.69 (t, J= 5.5 Hz, 1H), 5.97 (d, J= 6.0 Hz, 1H), 6.80-6.82 (m, 4H), 7.18-7.32 (m, 13H), 7.41-7.43 (m, 2H), 7.80 (s, 1H), 8.51 (s, 1H), 9.46 (s, 1H).

### Reference Example 3

### Synthesis of 2'-O-(t-butyldimethylsilyl)-N²⁻(dimethylaminomethylene) -5'-O- (4,4'-dimethoxytrityl)guanosine 3'-O-(2-cyanoethyl N,N-diisopropylphosphoroamidite) (Compound [14])

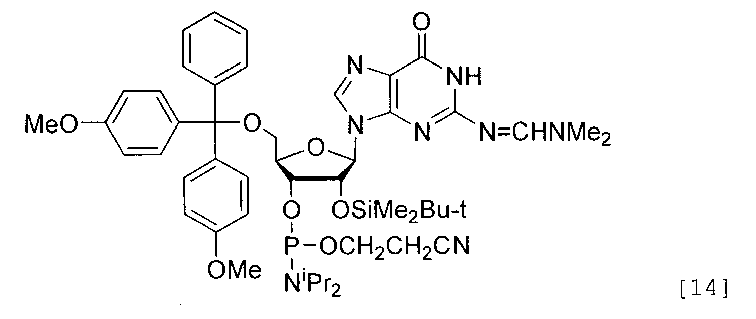

To a solution of 3.8 g (5 mmol) of 2'-O-(t-butyldimethylsilyl)-N²-(dimethylaminomethylene)-5'-0-(4,4'-dimethoxytrityl)guanosine (Compound [13]), 4.4 g (4.8 mL, 36 mmol) of 2,4,6-collidine, and 205 mg (0.2 mL, 2.5 mmol) of N-methylimidazole in 25 mL of THF, 3.0 g (12.5 mmol) of 2-cyanoethyl N,N-diisopropylchlorophosphoroamidite was added under ice-cooling, and the mixture was stirred at room temperature for 1 hour. Ethyl acetate was added to the mixture, and the mixture was washed with water, a saturated aqueous sodium bicarbonate solution and saturated brine and then concentrated under reduced pressure. The residue was dissolved in 20 mL of dichloromethane, and the solution was added dropwise to 1 L of hexane. The produced precipitate was filtered and dried under reduced pressure to obtain 3.7 g of the title compound (purity: about 90%) as a white powder. Yield: 78%.
¹H NMR (CDCl₃) δ -0.13, -0.10, 0.02, 0.03 (4s, 6H), 0.81, 0.82 (2s, 9H), 1.16-1.30 (m, 12H), 2.24-2.39 (m, 2H), 2.95, 3.07, 3.08, 3.09 (4s, 6H), 3.24-3.28 (m, 1H), 3.53-3.64 (m, 4H), 3.78, 3.79, 3.80, 3.81 (4s, 6H), 4.27-4.39 (m, 2H), 4.67-4.74 (m, 1H), 5.97, 6.04 (2d, J= 6.4 Hz, 1H), 6.71-6.85 (m, 4H), 7.21-7.48 (m, 13H), 7.85, 7.88 (2s, 1H), 8.50, 8.58 (s, 1H), 8.67, 8.69 (2s, 1H); ³¹H NMR (CDCl₃) δ 148.88, 150.02.

### Example 5

### Synthesis of 2'-O-(t-butyldimethylsilyl)-N²⁻(dimethylaminomethylene)guanosine 3'-O-(allyl 2-cyanoethylphosphate) (Compound [15])

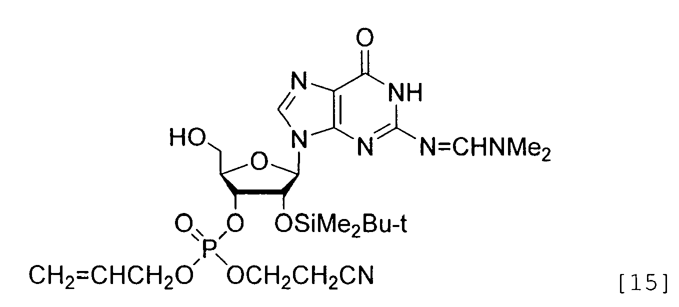

To a solution of 3.82 g (4.0 mmol) of 2'-O-(t-butyldimethylsilyl)-N²-(dimethylaminomethylene)-5'-0-(4,4'-dimethoxytrityl)guanosine 3'-O-(2-cyanoethyl N,N-diisopropylphosphoroamidite) (Compound [14]) and 200 mg of Molecular Sieves 3A in 16 mL of dry acetonitrile, 0.33 mL (279 mg, 4.8 mmol) of allyl alcohol was added, and the mixture was stirred at room temperature for 30 minutes. Then, 1.35 g (8.0 mmol) of imidazolium perchlorate was added thereto, and the mixture was stirred for 30 minutes. A 6.7% solution of 2-butanone peroxide/dimethyl phthalate in 8 mL of toluene was further added to the mixture, and the mixture was stirred for another 5 minutes. After removing Molecular Sieves 3A by filtration, ethyl acetate was added, and the mixture was washed with a saturated aqueous sodium bicarbonate solution and saturated brine, dried and concentrated under reduced pressure. The residue was dissolved in 30 mL of dichloromethane, 6.6 mL (10.4 g, 80 mmol) of dichloroacetic acid was added dropwise to the solution under stirring with ice-cooling, and the mixture was stirred for another 5 minutes. The reaction solution was added dropwise to 100 mL of a saturated aqueous sodium bicarbonate solution, the organic layer was separated, and the aqueous layer was extracted with dichloromethane. The organic layer was combined, and the mixture was dried over sodium sulfate and then concentrated under reduced pressure. The concentrated residue was purified by silica gel chromatography [silica gel: 100 g, methanol:dichloromethane (1:20) to (1:10)] to obtain 2.15 g of the title compound as a colorless amorphous material. Yield: 86%.
¹H NMR (CDCl₃) δ -0.27, -0.23 (2s, 3H), -0.09 (s, 3H), 0.78, 0.79 (2s, 9H), 2.80 (t, J= 6.0 Hz, 2H), 3.12 (s, 3H), 3.19 (s, 3H), 3.76 (q, J= 11.6 Hz, 1H), 3.88-3.91 (m, 1H), 4.27-4.35 (m, 2H), 4.46-4.48 (m, 1H), 4.63-4.67 (m, 2H), 4.97 (m, 1H), 5.08-5.17 (m, 1H), 5.31-5.37 (m, 1H), 5.41-5.45 (m, 1H), 5.69-5.72 (m, 1H), 5.95-6.03 (m, 1H), 7.63 (s, 1H), 8.40 (s, 1H), 9.37 (br, 1H) ; ³¹H NMR (CDCl₃) δ -2.34; HRMS (ESI⁺) calcd for C₂₅H₄₀N₇O₈PSi⁺ (M+H⁺) m/z 626.2518, found m/z 626.2628.

### EXAMPLE 6

### Synthesis of 2-cyanoethyl [2'-0-(t-butyldimethylsilyl)-N²-(dimethylaminomethylene)guanylyl](3'→5')[2'-O-(t-butyldimethylsilyl)-N²-(dimethylaminomethylene)guanosine 3'-O-(allyl 2-cyanoethylphosphate)] (Compound [16])

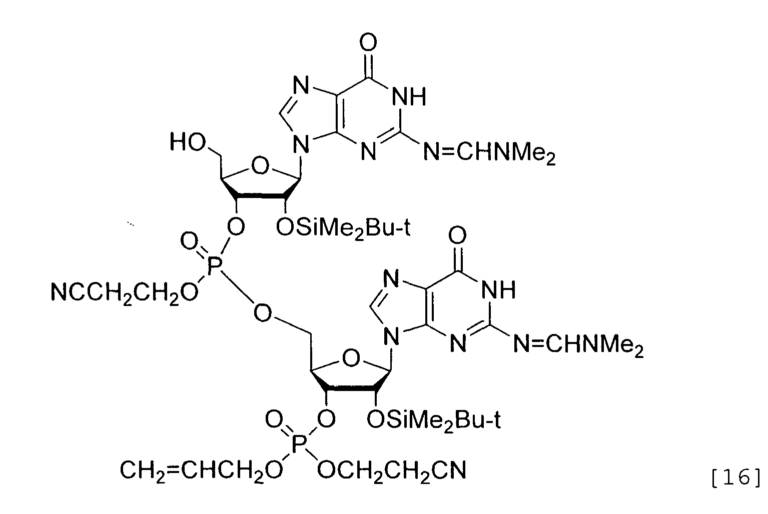

A solution of 1.7 g (1.6 mmol) of 2'-O-(t-budyldimethylsilyl)-N²-(dimethylaminomethylene)-5'-O-(4,4'-dimethoxytrityl)guanosine 3'-O-(2-cyanoethyl N,N-diisopropylphosphoroamidite), 0.98 g (1.6 mmol) of Compound [15] and 0.2 g of Molecular Sieves 3A in 10 mL of dry acetonitrile was stirred at room temperature for 30 minutes, 0.54 g (3.2 mmol) of imidazolium perchlorate was added, and the mixture was stirred for 30 minutes. A 6.7% solution of 2-butanone peroxide/dimethyl phthalate in 3.2 mL of toluene was further added, and the mixture was stirred for another 5 minutes. After removing Molecular Sieves 3A by filtration, the mixture was concentrated under reduced pressure. The residue was dissolved in 20 mL of dichloromethane, 3.3 mL (5.2 g, 40 mmol) of dichloroacetic acid was added dropwise to the solution under stirring with ice-cooling, and the mixture was stirred for 5 minutes. The reaction solution was added dropwise to 100 mL of a saturated aqueous sodium bicarbonate solution, the organic layer was separated, and the aqueous layer was extracted with dichloromethane. The organic layer was combined, and the mixture was dried over sodium sulfate and then concentrated under reduced pressure. The concentrated residue was purified by silica gel chromatography (silica gel: 50 g, methanol:dichloromethane (1:20) to (1:10)) to obtain 1.53 g of the title compound (mixture of diastereomers) as a colorless amorphous material. Yield: 82%.
¹H NMR (CDCl₃) δ -0.26-0.03 (m, 12H), -0.74-0.96 (m, 18H), 2 . 72-2. 80 (m, 4H), 3.09, 3. 11, 3.22 (4s, 12H), 3.70-3.81 (m, 2H), 4.17-4.64 (m, 11H), 4.88-5.25 (m, 4H), 5.31-5.44 (m, 2H), 5.73-6.00 (m, 3H), 7.51-7.82 (m, 4H), 8.37 (s, 1H), 8.61 (s, 1H) , 9.24 (br, 2H); ³¹P NMR (CDCl₃) δ -1.78, -1.72, -1.45, -1.32, -1.04, -0.83; HRMS (ESI⁺) calcd for C₄₇H₇₄N₁₄O₁₅P₂Si₂⁺ (M+H⁺) 1193.4545, found 1193.5971.

### EXAMPLE 7

### Synthesis of cyclic bis(3'→5')bis[2'-O-(t-butyldimethylsilyl)-N²-(dimethylaminomethylene)guanylic acid] bis(2-cyanoethyl) ester (Compound [17])

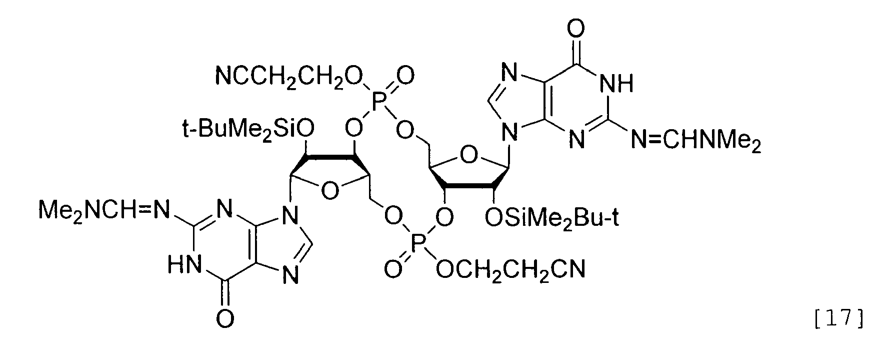

To a solution of 848 mg (0.71 mmol) of Compound [16] in 10 mL of acetone, 1.06 g (7.1 mmol) of sodium iodide was added, and the mixture was heated under reflux for 2 hours. The precipitate was recovered by filtration, washed with 50 mL of cold acetone and dried. The obtained powder was suspended in THF, and 0.11 mL (115 mg, 1.4 mmol) of N-methylimidazole and 424 mg (1.0 mmol) of triisopropylbenzenesulfonyl chloride were added to the suspension. The mixture was stirred at room temperature for 36 hours. Water was added to the reaction solution, and the mixture was stirred for 1 hour and then extracted with ethyl acetate. The residue obtained by concentration under reduced pressure was purified by silica gel chromatography (silica gel: 50 g, methanol:dichloromethane (1:20) to (1:10)) to obtain 676 mg of the title compound as a colorless amorphous material. Yield: 85%.
¹H NMR (CD₃OD) δ -0.14 (s, 6H), 0.09 (s, 6H), 0.76 (s, 18H), 2.95 (t, J= 6.0 Hz, 4H), 3.14 (s, 6H), 4.13-4.21 (m, 2H), 4.35- 4.68 (m, 10H), 5.36, 5.38 (2d, J= 5.0 Hz, 2H), 5.31-5.44 (m, 2h), 5.91- 5.96 (m, 4H), 7.08 (s, 2H), 7.94 (s, 2H), 8. 69 (s, 2H); ³¹P NMR (CD₃OD) δ 1.02; HRMS (ESI⁺) calcd for C₄₄H₆₉N₁₄O₁₄P₂Si₂⁺ (M+H⁺) 1135.4126, found 1135.4455.

### EXAMPLE 8

### Synthesis of cyclic bis(3'→5')diguanylic acid (Compound [11])

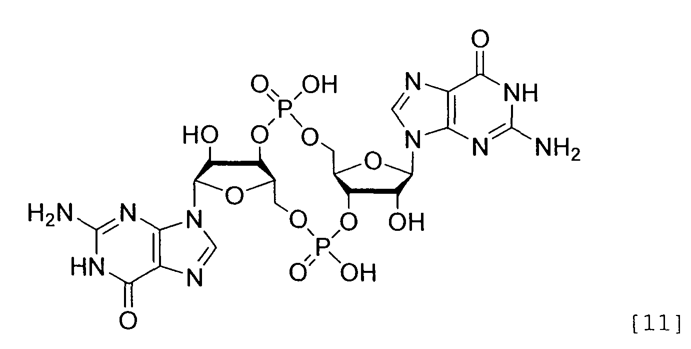

To a solution of 116 mg (0.1 mmol) of Compound [17] in 8 mL of methanol, 8 mL of aqueous ammonia was added, and the solution was stirred at 50°C for 12 hours. The reaction solution was concentrated and dried under reduced pressure. To the obtained residue, 1.0 mL of a triethylamine/3HF complex was added and the mixture was stirred at room temperature for 12 hours. 10 mL of a 1 M ammonium acetate buffer was added to the reaction solution, and the mixture was stirred at 30 to 40°C. The precipitate was removed, and the obtained solution was purified by HPLC to obtain 67 mg of the title compound. Yield: 89%.
[HPLC conditions]
Column: COSMOSIL 5C₁₈-AR-300 (25φ x 200 mm)
Eluent: Liquid A, 1 mM aqueous solution of ammonium acetate; Liquid B, 0.2 mM ammonium acetate/water-acetonitrile (20:80) solution

| Gradient conditions: | | | | |
|---|---|---|---|---|
| Time (min) | 0 | 8 | 55 | 63 |
| B% | 0 | 0 | 60 | 100 |

Detection wavelength: 254 nm, Flow rate: 10 mL/min

### Industrial Applicability

The synthesis method of the invention is useful as an industrial preparative method for synthesizing a cyclic bis(3'→5')dinucleotides with high efficiency. Furthermore, the cyclic bis(3'→5') dinucleotide obtained by the synthesis method of the invention is useful as a medicine such as an anticancer agent.

## Claims

1. A method for synthesizing a compound represented by Formula [2]: 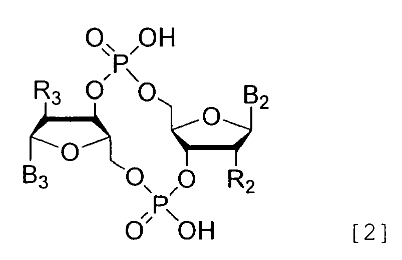 wherein R₂ and R₃ each independently represent a hydrogen atom, a halogen atom, a methoxy group, a 2-methoxyethoxy group or a hydroxyl group; and B₂ and B₃ each independently represent a nucleic acid base,
or a salt thereof from a compound represented by Formula [1]: 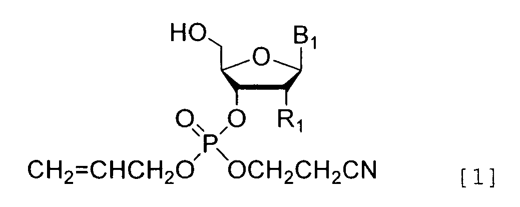 wherein R₁ represents a hydrogen atom, a halogen atom, a methoxy group, a 2-methoxyethoxy group, or a hydroxyl group substituted with a hydroxyl protective group; and B₁ represents a nucleic acid base which may be protected.

2. A method for synthesizing a compound represented by Formula [2]: 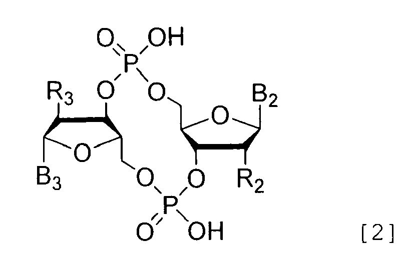 wherein R₂, R₃, B₂ and B₃ have the same meanings as defined for R₂, R₃, B₂ and B₃ of Formula [2] in claim 1 above,
or a salt thereof from a compound represented by Formula [3]: 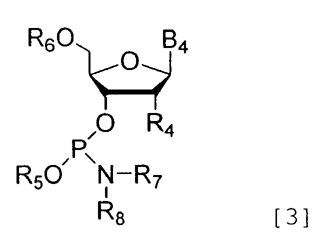 wherein R₄ represents a hydrogen atom, a halogen atom, a methoxy group, a 2-methoxyethoxy group, or a hydroxyl group substituted with a hydroxyl protecting group; B₄ represents a nucleic acid base which may be protected; R₅ represents an allyl group or a 2-cyanoethyl group; R₆ represents a hydroxyl protecting group; and R₇ and R₈ each independently represent an alkyl group having 1 to 4 carbon atoms, or R₇ and R₈ may be bonded to form a ring containing a nitrogen atom,
or a compound represented by Formula [4]: 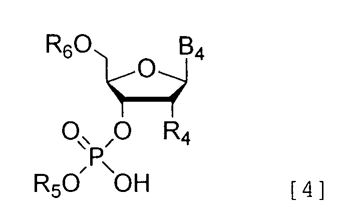 wherein R₄, R₅, R₆ and B₄ have the same meanings as defined for R₄, R₅, R₆ and B₄ of Formula [3] above,
and from a compound represented by Formula [1]: 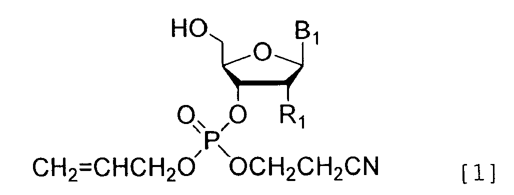 wherein R₁ and B₁ have the same meanings as defined for R₁ and B₁ of Formula [1] in claim 1 above.

3. The method according to claim 1 or 2, wherein the synthetic intermediate is a compound represented by Formula [5]: 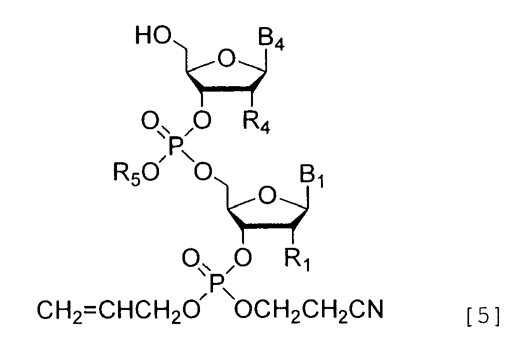 wherein R₁ and R₄ each independently represent a hydrogen atom, a halogen atom, a methoxy group, a 2-methoxyethoxy group, or a hydroxyl group substituted with a hydroxyl protecting group; B₁ and B₄ each independently represent a nucleic acid base which may be protected; and R₅ is an allyl group or a 2-cyanoethyl group.

4. The method according to claim 1 or 2, wherein the synthetic intermediate is a compound represented by Formula [6]: 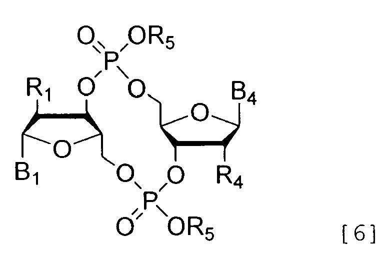 wherein R₁, R₄, R₅, B₁ and B₄ have the same meanings as defined for R₁, R₄, R₅, B₁ and B₄ of Formula [5] in the previous claim.

5. The method according to claim 1, wherein with respect to Formula [1], R₁ is a hydrogen atom, a fluorine atom, a methoxy group, a 2-methoxyethoxy group or a t-butyldimethylsilyloxy group; and with respect to Formula [2], R₂ and R₃ each independently represent a hydrogen atom, a fluorine atom, a methoxy group, a 2-methoxyethoxy group or a hydroxyl group.

6. The method according to claim 2, wherein with respect to Formulas [1], [3] and [4], R₁ and R₄ each independently represent a hydrogen atom, a fluorine atom, a methoxy group, a 2-methoxyethoxy group or a t-butyldimethylsilyloxy group; and with respect to Formula [2], R₂ and R₃ each independently represent a hydrogen atom, a fluorine atom, a methoxy group, a 2-methoxyethoxy group or a hydroxyl group.

7. A compound represented by Formula [1]: 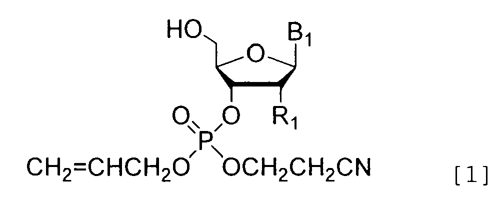 wherein R₁ has the same meaning as defined for R₁ of Formula [1] in claim 1 above; and B₁ represents a nucleic acid base which may be protected.

8. A compound represented by Formula [5]: 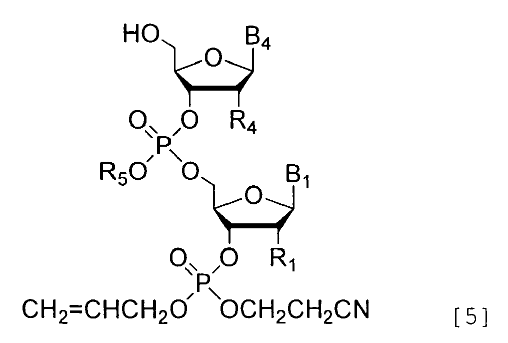 wherein R₁, R₄, R₅, B₁ and B₄ have the same meanings as defined for R₁, R₄, R₅, B₁ and B₄ of Formula [5] in claim 3 above.

9. A compound represented by Formula [6]: 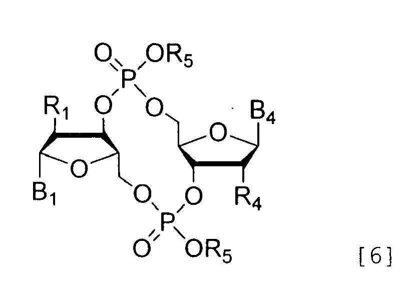 wherein R₁, R₄, R₅, B₁ and B₄ have the same meanings as defined for R₁, R₄, R₅, B₁ and B₄ of Formula [6] in claim 4 above.
